# EUROPEAN PATENT APPLICATION

(11) **EP 4 617 374 A2**
(43) Date of publication of application: **17.09.2025**
(21) Application number: 25176809.9
(22) Date of filing: 11.10.2016
(51) Int. Cl.: C12Q 1/68

(54) **DIAGNOSTIC ASSAY FOR URINE MONITORING OF BLADDER CANCER**

(30) Priority: 08.10.2015 US 201562239202 P
(62) Divisional of application: 16854568.9
(71) Applicant: Convergent Genomics, Inc., San Francisco, CA 94107 (US); Levin, Trevor, San Francisco, CA 94107 (US); King, Carly, San Francisco, CA 94107 (US); Phillips, Kevin, San Francisco, CA 94107 (US); Evans-Holm, Martha, San Francisco, CA 94107 (US); Li, Yueyao, San Francisco, CA 94107 (US)
(72) Inventor: LEVIN, Trevor, San Francisco, CA 94107 (US); KING, Carly, San Francisco, CA 94107 (US); PHILIPS, Kevin, San Francisco, CA 94107 (US); EVANS-HOLM, Martha, San Francisco, CA 94107 (US); LI, Yueyao, San Francisco, CA 94107 (US)
(74) Representative: Mewburn Ellis LLP

(57) **Abstract**

An improved diagnostic assay and methods relating to the same that are directed to mutation focused disease diagnosis and surveillance biomarker panels wherein potential genomic regions are selected based on their ability to encompass the genomic diversity of a patient population, maximize the number of unique markers monitored within each patient are maximized while balancing these factors with empirical sequencing performance, geographic clustering of events with a region across diverse patients, and size and cost associated with measuring the respective genomic region. The methods also include quality control steps to reduce noise and maximize the presence of relevant markers.

## Description

### FIELD OF THE INVENTION

The disclosure herein pertains to the identification of cancer, and more particularly to the detection, prognosis, diagnosis and treatment of bladder cancer of an individual or group of individuals through genetic biomarkers and improved methodologies in gene sequencing and analysis.

### BACKGROUND OF THE INVENTION

Bladder cancer is projected to be the sixth most common solid cancer in North America, with estimates of more than 74,000 new cases in the US in 2014 (American Cancer Society, "Cancer Facts & Figures 2014." 2014). Diagnosis is usually made following symptoms of painless hematuria (i.e., blood in the urine) that triggers a visit to a physician. Common risk factors for bladder cancer include smoking, race (higher incidence in Caucasian, lower incidence in Asians), occupational exposure, and gender (bladder cancer is the 4th most common cancer in men but 11th in women). Roughly two-thirds of all bladder cancers will present as superficial disease, with invasive disease presenting in the remaining third.

Despite improvements in surgical and medical management of superficial bladder cancer, roughly 70-80% of bladder cancers recur following initial treatment and 10-20% of early stage disease will progress to invasion of the bladder wall ((H. W. Herr, J. R. Faulkner, H. B. Grossman, R. B. Natale, R. deVere White, M. F. Sarosdy, and E. D. Crawford, "Surgical Factors Influence Bladder Cancer Outcomes: A Cooperative Group Report," J. Clin. Oncol., vol. 22, no. 14, pp. 2781-2789, Jul. 2004); ("Bladder Cancer Treatment (PDQ^{®})," *National Cancer Institute.* www.cancer.gov/cancertopics/pdq/treatment/bladder/HealthProfessional/page1. [Accessed: 02-Dec-2014]); and (American Urological Association, "Guideline for the Management of Nonmuscle Invasive Bladder Cancer: (Stages Ta, T1 and Tis: Update (2007)." 2007)). As a result, patients with superficial disease treated by trans-urethral resection undergo a rigorous screening protocol, with regular cystoscopies to assess recurrence of the disease every 3-6 months for at least five years and annually thereafter ((American Urological Association, "Guideline for the Management of Nonmuscle Invasive Bladder Cancer: (Stages Ta, T1 and Tis: Update (2007)." 2007); and (National Comprehensive Cancer Network, "NCCN Clinical Practice Guidelines in Oncology (NCCN Guidelines): Bladder Cancer," Version 2.2014)). Screening by cystoscopy is highly invasive for patients, requiring a scope to be inserted into the bladder through the urethra, and is thus associated with screening non-compliance in up to 60% of patients (D. Schrag, L. J. Hsieh, F. Rabbani, P. B. Bach, H. Herr, and C. B. Begg, "Adherence to Surveillance Among Patients With Superficial Bladder Cancer," J. Natl. Cancer Inst., vol. 95, no. 8, pp. 588-597, Apr. 2003). Furthermore, as these procedures must be carried out by a urologist, and follow up is frequent and life-long, the costs involved in management of bladder cancer are significant, resulting in the highest average life-long per-patient surveillance cost of any solid cancer (M. F. Botteman, C. L. Pashos, A. Redaelli, B. Laskin, and R. Hauser, "The health economics of bladder cancer: a comprehensive review of the published literature," PharmacoEconomics, vol. 21, no. 18, pp. 1315-1330, 2003). A lack of sufficiently sensitive and specific urine based assays for detection of bladder cancer recurrence is a significant unmet medical need.

### SUMMARY OF THE INVENTION

The present embodiments address the needs discussed above by developing an improved assay and related sample preservation and processing methods for bladder cancer diagnosis, and including a urine nucleic acid sequencing diagnostic for sensitive and specific detection of bladder cancer. Based on data from urine and tumor samples from human patients with bladder cancer, the improved assay presents a high value, cost effective clinical diagnostic assay.

Large scale cancer genome initiatives have significantly advanced our understanding of the genomic events associated with bladder cancer. With recent completion of The Cancer Genome Atlas project for urothelial cancers, a comprehensive list of the most common mutations in bladder cancer is available, including known genes such as *TP53, PIK3CA, RB1,* and *FGFR3* (The Cancer Genome Atlas Research Network, "Comprehensive molecular characterization of urothelial bladder carcinoma," Nature, vol. 507, no. 7492, pp. 315-322, Mar. 2014). A number of other publications have identified additional mutations in bladder cancer, some of which are of particular interest since they show the presence of mutations in early stage or low grade tumors (Table 1). For example, *TERT* promoter mutations have been found to be very common in early stage invasive bladder cancer ((C. D. Hurst, F. M. Platt, and M. A. Knowles, "Comprehensive Mutation Analysis of the TERT Promoter in Bladder Cancer and Detection of Mutations in Voided Urine," Eur. Urol.); (P. J. Killela, Z. J. Reitman, Y. Jiao, C. Bettegowda, N. Agrawal, L. A. Diaz, A. H. Friedman, H. Friedman, G. L. Gallia, B. C. Giovanella, A. P. Grollman, T.-C. He, Y. He, R. H. Hruban, G. I. Jallo, N. Mandahl, A. K. Meeker, F. Mertens, G. J. Netto, B. A. Rasheed, G. J. Riggins, T. A. Rosenquist, M. Schiffman, I.-M. Shih, D. Theodorescu, M. S. Torbenson, V. E. Velculescu, T.-L. Wang, N. Wentzensen, L. D. Wood, M. Zhang, R. E. McLendon, D. D. Bigner, K. W. Kinzler, B. Vogelstein, N. Papadopoulos, and H. Yan, "TERT promoter mutations occur frequently in gliomas and a subset of tumors derived from cells with low rates of self-renewal," Proc. Natl. Acad. Sci., vol. 110, no. 15, pp. 6021-6026, Apr. 2013); (X. Liu, G. Wu, Y. Shan, C. Hartmann, A. von Deimling, and M. Xing, "Highly prevalent TERT promoter mutations in bladder cancer and glioblastoma," Cell Cycle, vol. 12, no. 10, pp. 1637-1638, May 2013); and (I. Kinde, E. Munari, S. F. Faraj, R. H. Hruban, M. Schoenberg, T. Bivalacqua, M. Allaf, S. Springer, Y. Wang, L. A. Diaz, K. W. Kinzler, B. Vogelstein, N. Papadopoulos, and G. J. Netto, "TERT promoter mutations occur early in urothelial neoplasia and are biomarkers of early disease and disease recurrence in urine," Cancer Res., vol. 73, no. 24, pp. 7162-7167, Dec. 2013)) Similarly, mutations in *FGFR3* have long been known to be common in early stage, non-invasive bladder cancers and *STAG2* mutations have recently been identified to have a similar pattern ((C. Billerey, D. Chopin, M. H. Aubriot-Lorton, D. Ricol, S. Gil Diez de Medina, B. Van Rhijn, M. P. Bralet, M. A. Lefrere-Belda, J. B. Lahaye, C. C. Abbou, J. Bonaventure, E. S. Zafrani, T. van der Kwast, J. P. Thiery, and F. Radvanyi, "Frequent FGFR3 mutations in papillary non-invasive bladder (pTa) tumors," Am. J. Pathol., vol. 158, no. 6, pp. 1955-1959, Jun. 2001); (C. F. Taylor, F. M. Platt, C. D. Hurst, H. H. Thygesen, and M. A. Knowles, "Frequent inactivating mutations of STAG2 in bladder cancer are associated with low tumour grade and stage and inversely related to chromosomal copy number changes," Hum. Mol. Genet., vol. 23, no. 8, pp. 1964-1974, Apr. 2014); (D. A. Solomon, J.-S. Kim, J. Bondaruk, S. F. Shariat, Z.-F. Wang, A. G. Elkahloun, T. Ozawa, J. Gerard, D. Zhuang, S. Zhang, N. Navai, A. Siefker-Radtke, J. J. Phillips, B. D. Robinson, M. A. Rubin, B. Volkmer, R. Hautmann, R. Küfer, P. C. W. Hogendoorn, G. Netto, D. Theodorescu, C. D. James, B. Czerniak, M. Miettinen, and T. Waldman, "Frequent truncating mutations of STAG2 in bladder cancer," Nat. Genet., vol. 45, no. 12, pp. 1428-1430, Dec. 2013)). The sum of these and similar investigations have been utilized by the inventors to design an assay that comprehensively surveys the entire spectrum of mutations in both low and high grade bladder cancer with high sensitivity.

Over the past several years, highly sensitive next generation sequencing (NGS) techniques have emerged as a powerful way to examine cancer biomarkers. While these technologies routinely permit broad sequencing of tumors to identify mutations abundant at 5% or greater frequency within a population, standard methods and machine and assay noise typically does not permit de novo identification of mutations below 1-5% allele frequency. Further, most tumor sequencing approaches are dependent on sequencing of matched normal tissue from a patient to screen out SNPs (single nucleotide polymorphisms) or non-pathogenic variations in their genome.

Certain present embodiments improve upon this approach with an expanded panel of DNA based markers which more fully encompasses the genomic diversity of bladder cancer. The low coefficients of variation and high sensitivity afforded by these novel approaches permit technical sensitivity comparable to other high sensitivity clinical platforms for measurement of nucleic acid mutations. In light of these improvements in sensitivity, the inventors have utilized NGS to provide un-paralleled ability to measure truly tumor intrinsic markers in a personalized manner over the course of a patient's treatment or recurrence surveillance. As used herein, NGS includes a number of different modern sequencing technologies including: Illumina (Solexa) sequencing, Roche 454 sequencing, Ion torrent: Proton / PGM sequencing and SOLiD sequencing. These technologies allow the rapid sequencing of DNA and RNA than the previously used Sanger sequencing techniques.

The inventors have utilized NGS' potential for minimally invasive detection and monitoring of cancer, and enabling simultaneous revealing of underlying abnormalities in tumor suppressor and promoter genes that drive the cancer. This valuable insight has allowed tracking of tumor evolution over the course of treatment and recurrence, and which changes may correspond with progression risk, therapeutic response, and time to recurrence.

In exemplary embodiments, analysis algorithms are implemented in the improved assay that allow longitudinal monitoring of urine DNA following an initial assessment of a patient's primary tumor nucleic acid. By developing an enhanced targeted panel of biomarkers that are capable of encompassing the genomic and clinical diversity of bladder cancer, and eventually hematuria, the method of certain present embodiments provide high technical performance while simultaneously achieving clinically feasible assay costs and processing times. The methods provide the opportunity to monitor the urine of bladder patients in a manner that will likely yield much higher sensitivity and specificity than existing techniques and provides advantages over existing FDA approved urine assays.

Certain of present embodiments provide a method for detecting mutations in one or more genes associated with bladder cancer. These methods involve isolating nucleic acid, DNA or RNA, from a urine sample from a subject, and analyzing the nucleic acid to obtain nucleic acid sequence data suitable to detect presence or absence of one or more mutations in one or more of genes associated with bladder cancer. Optionally, the isolated nucleic acid is analyzed for epigenetic markers such as 5-methylcytosine methylation, CpG islands, or other variations upon nucleic acid structure. Optionally, the isolated nucleic acid is cell-free nucleic acid and nucleic acid isolated from cells in the urine sample.

Mutation as used herein includes, without limitation, the deletion or duplication of a gene or a portion of a gene, translocation or fusion of a gene or a portion of a gene, deletions and duplications of a whole chromosome or a portion of a chromosome, an indel or a single point mutation.

Certain present embodiments provide methods for prognosing bladder cancer in a subject. Embodiments involve determining the presence or absence of at least one mutation or epigenetic alteration in at least one gene associated with bladder cancer in a nucleic acid sample obtained from the urine of a subject, or a genotype dataset derived from the subject, where the presence and/or relative abundance of the at least one mutation or epigenetic alteration in the at least one gene is indicative of bladder cancer prognosis.

Certain of the present embodiments provide methods for diagnosing bladder cancer in a subject. Embodiments involve determining the presence or absence of at least one mutation in at least one gene associated with bladder cancer in a nucleic acid sample obtained from the urine of the subject, or a genotype dataset derived from the subject. In an exemplary embodiment, the presence and/or relative abundance of the at least one mutation or the at least one epigenetic alteration in the at least one gene is indicative of bladder cancer. In an exemplary embodiment, the subject presents with blood in their urine. In another exemplary embodiment, the subject is asymptomatic or otherwise believed to be a healthy individual. In another embodiment, the subject is a high risk individual or population of individuals, such as cigarette smokers, individuals with histories of occupational carcinogen exposures, individuals with histories of drinking water from wells or ground water contaminated with arsenic or other suspected carcinogens, or individuals living within geographical cancer hotspots.

Certain of the present embodiments determine susceptibility of a subject to bladder cancer comprising determining the presence or absence of at least one or more mutations associated with bladder cancer in at least one or more genes in a genotype dataset derived from an individual or subject, where determination of the presence and/or relative abundance of the at least one mutation is indicative of increased susceptibility to bladder cancer in the subject.

In an exemplary embodiment, the genotype dataset includes information about the allelic status of the individual, i.e., information about the identity of the two alleles carried by the individual for the mutations associated with bladder cancer. The genotype dataset may comprise allelic information about one or more mutations or epigenetic marker, including two or more mutations or epigenetic marker, three or more mutations or epigenetic marker, five or more mutations or epigenetic marker, one hundred or more mutations or epigenetic marker, etc. In some embodiments, the genotype dataset includes genotype information from a whole-genome assessment of the individual that may include hundreds of thousands of mutations, or even one million or more mutations.

In certain embodiments, determination of a susceptibility includes comparing the nucleic acid sequence data to a database containing correlation data between the at least one mutation and/or epigenetic marker and susceptibility to bladder cancer. In some embodiments, the database includes at least one risk measure of susceptibility to bladder cancer for the at least one mutation and/or epigenetic markers. The sequence database can for example be provided as a look-up table that contains data that indicates the susceptibility of bladder cancer for any one, or a plurality of, particular mutations and/or epigenetic markers.

Certain of the present embodiments provide a method for monitoring bladder cancer progression or recurrence of bladder cancer in a subject. The method involves obtaining a first and second sample of urine, at different points in time, from the subject having cancer, isolating nucleic acid from urine sample, and/or analyzing the nucleic acid to obtain nucleic acid sequence data suitable to detect presence or absence of one or more mutations and/or epigenetic markers in one or more of genes associated with bladder cancer. The method further involves comparing the presence or absence of the one or more mutations and/or epigenetic markers detected in the first sample to the presence or absence of the one or more mutations and/or epigenetic markers detected in the second sample. This approach harnesses unique advantages by allowing algorithms implemented to compare results between samples collected serially from the same patient at different times, thereby enhancing assay sensitivity and specificity while also personalizing recurrence monitoring for each patient and allowing the assay to distinguish between biological recurrence of the primary tumor and emergence of divergent multi-foci disease.

In certain embodiments, the at least one mutation and/or epigenetic alteration in at least one gene is selected from the mutations listed in Table 1.

Certain embodiments also provide computer-implemented aspects. In one such aspect, an embodiment provides a computer-readable medium having computer executable instructions for determining susceptibility to bladder cancer in a subject, the computer readable medium including: data representing at least one mutation and/or epigenetic marker; and a routine stored on the computer readable medium and adapted to be executed by a processor to determine susceptibility to bladder cancer in an individual based on the one or more mutations and/or epigenetic alteration of at least one or more genes in the subject.

Certain embodiments further provide an apparatus for determining an indicator for bladder cancer in a subject, including: a processor, a computer readable memory having computer executable instructions adapted to be executed on the processor to analyze mutation or gene information for at least one subject with respect to bladder cancer, and generate an output based on the mutation or genetic information. The output may include an information or a risk measure of the at least one mutation and/or epigenetic alteration as an indicator of bladder cancer for the subject.

In an exemplary embodiment, the computer readable memory includes data indicative of the frequency of at least one mutation and/or epigenetic alteration of at least one gene in a plurality of individuals diagnosed with bladder cancer. The memory can also include data indicative of the frequency of the at least one mutation and/or epigenetic alteration of at least one gene in a plurality of reference individuals. A risk measure can be based on a comparison of the at least one mutation and/or epigenetic alteration and/or a genotype data set status for the subject to the data indicative of the frequency of the at least one mutation and/or genotype data set information for the plurality of individuals diagnosed with bladder cancer.

In an alternative embodiment, the computer readable memory further includes data indicative of the risk of developing bladder cancer associated with at least one mutation and/or epigenetic alteration of at least one gene or at least one genotype data set. A risk measure for the subject can be based on a comparison of the genotype data set for the subject to the risk associated with the at least one mutation and/or epigenetic alteration of the at least one gene or the at least one genotype data set.

In another embodiment, the computer readable memory further includes data indicative of the frequency of at least one mutation and/or epigenetic alteration of at least one gene or at least one genotype data set in a plurality of individuals diagnosed with bladder cancer. The memory can also include data indicative of the frequency of at the least one mutation and/or epigenetic alteration of at least one gene or at least one genotype data set in a plurality of reference individuals. Here, the risk of developing bladder cancer can be based on a comparison of the frequency of the at least one mutation and/or epigenetic alteration or genotype data set in individuals diagnosed with bladder cancer, and reference individuals. In a certain embodiment, the at least one mutation or epigenetic alteration is selected from those set forth in Table 1.

Certain embodiments also relate to kits. In one such aspect, an embodiment relates to a kit for assessing susceptibility to bladder cancer in a subject, the kit comprising reagents necessary for selectively detecting at least one mutation and/or epigenetic alteration of at least one gene associated with bladder cancer in the genome of the subject, where the presence of the at least one mutation and/or epigenetic alteration is indicative of increased susceptibility to bladder cancer. In an exemplary embodiment, the kit further includes a collection of data including correlation data between the at least one mutation and susceptibility to bladder cancer. The correlation data may be in any suitable formation, for example as a Relative Risk measure (RR), odds ratio (OR), or other convenient measure known to the skilled person. In one embodiment, the collection of data is on a computer-readable medium.

In another aspect, an embodiment relates to a kit for assessing susceptibility to bladder cancer in a subject, the kit comprising reagents for selectively detecting at least one mutation and/or epigenetic alteration of at least one gene in the genome of the subject, wherein the mutation is selected and wherein the presence of the at least one mutation and/or epigenetic alteration is indicative of a susceptibility to bladder cancer. In one embodiment, the at least one mutation and/or epigenetic alteration is selected from those set forth in Table 1.

Kit reagents are used in certain embodiments. In one embodiment such reagents include at least one contiguous oligonucleotide that hybridizes to a fragment of the genome of the individual including the at least one mutation. In another embodiment, the kit includes at least one pair of oligonucleotides that hybridize to opposite strands of a genomic segment obtained from the subject, where each oligonucleotide primer pair is designed to selectively amplify a fragment of the genome of the individual that includes one mutation and/or epigenetic alteration. The mutation and/or epigenetic alteration can be selected from the group consisting of the mutations and/or epigenetic alteration as defined in Table 1. In one exemplary embodiment, the oligonucleotide is completely complementary to the genome of the individual. In another exemplary embodiment, the kit further contains buffers and enzymes for amplifying the segment. In another exemplary embodiment, the reagents further include a label for detecting the fragment.

Kits according to certain present embodiments are also used in the other methods of the embodiments, including methods of assessing risk of developing at least a second primary tumor in a subject previously diagnosed with bladder cancer, methods of assessing a subject for probability of response to a bladder cancer therapeutic agent, methods of assessing a subject for probability of disease pathologic stage or grade progression, and methods of monitoring progress of a treatment of a subject diagnosed with bladder cancer and given a treatment for the disease.

Other objects, features and advantages of certain embodiments will become apparent from the following detailed description. It should be understood, however, that the detailed description and the specific examples, while indicating specific embodiments, are given by way of illustration only, since various changes and modifications within the spirit and scope of the inventive embodiments will become apparent to those skilled in the art from this detailed description.

### BRIEF DESCRIPTION OF THE DRAWINGS

These and other features, aspects, and advantages of the present embodiments will become better understood with reference to the following description and appended claims, and accompanying drawings where:
Fig. 1 is a matrix displaying the preliminary next generation sequencing panel which encompasses the biologic diversity of 96% of bladder cancer patients.
Fig. 2 depicts graphical representations of the respective noise level in the RAD51 gene before and after the application of error suppression methods and high efficiency library conversion.
Fig. 3 shows a line dilution graph wherein such line or dilution is a unique urine reference DNA sample diluted into another urine DNA reference sample using standard commercially available methods.
Fig. 4 shows line dilution graph wherein such line or dilution is a unique DNA sample diluted into a reference DNA sample after disclosed quality control measures have been implemented.
Fig 5 illustrates a flow algorithm comprising genomic libraries and raw patient sequencing data that serve as inputs into a metric generating algorithm, mutation calling algorithm and clinical reporting algorithms.
Fig 6 illustrates (A) a computational platform that is supported by a data infrastructure (B) comprised of both proprietary (B i, ii, iv) and open source (B iii) genomic libraries.
Fig. 7 is a series of graphs showing in nucleic acid integrity and variation over time of day within the same individual.
Fig. 8 depicts a series of patient profiles depicting unique urine nucleic acid profiles.
Fig. 9 presents two graphs showing the relationship between allele frequency in tumor and urine nucleic acid in two patient matched samples where urine was collected while tumor was present in the bladder.
Fig. 10 presents a graph demonstrating post-filtering mutational abundance in non-cancer and cancer patients' urine nucleic acid

### DETAILED DESCRIPTION

In the description that follows, a number of terms are extensively utilized. In order to provide a clearer and consistent understanding of the specification and claims, including the scope to be given such terms, the following definitions are provided.

The use of the word "a" or "an" when used in conjunction with the term "comprising," "including," "having" or "containing," or other tenses thereof, in the claims and/or the specification may mean "one," but are also consistent with the meaning of "one or more," "at least one," and "one or more than one" or "a plurality."

Throughout the written description hereof (which includes the claims), the term "about" is used to indicate that a value includes the standard deviation of error for the device or method being employed to determine the value.

The use of the term "or" in the claims is used to mean either "and" or "or" ("and/or") unless explicitly indicated to refer to alternatives only or if alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

It also is specifically understood that any numerical value recited herein includes all values from the lower value to the upper value, inclusive of such values, and that all possible combinations of numerical values between the lowest value and the highest value enumerated are to be considered to be expressly stated in this written description (which includes the claims). For example, if a range is stated as 1% to 50%, it is intended that values such as 2% to 40%, 10% to 30%, or 1% to 3%, etc., are expressly enumerated in the specification and claims.

"Contacting" refers to the process of bringing into contact at least two distinct species such that they can react. It should be appreciated, however, the resulting reaction product can be produced directly from a reaction between the added reagents or from an intermediate from one or more of the added reagent which can be produced in the reaction mixture.

A "Single Nucleotide Polymorphism" or "SNP" is a DNA sequence variation occurring when a single nucleotide at a specific location in the genome differs between members of a species or between paired chromosomes in an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The SNP nomenclature as reported herein refers to the official Reference SNP (rs) ID identification tag as assigned to each unique SNP by the National Center for Biotechnological Information (NCBI).

"Nucleic acid," "oligonucleotide," and "polynucleotide" refer to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. The term nucleic acid is used interchangeably with gene, cDNA, and mRNA encoded by a gene.

The term "susceptibility", as described herein, refers to the proneness of an individual towards the development of a certain state (e.g., a certain trait, phenotype or disease, e.g., bladder cancer), or towards being less able to resist a particular state than the average individual. The term encompasses both increased susceptibility and decreased susceptibility. Thus, particular mutations in certain genes of certain embodiments as described herein may be characteristic of increased susceptibility (i.e., increased risk) of bladder cancer, as characterized by a relative risk (RR) or odds ratio (OR) of greater than one for the particular mutation, allele or haplotype. Alternatively, the mutations or combinations thereof of certain embodiments are characteristic of decreased susceptibility (i.e., decreased risk) of bladder cancer, as characterized by a relative risk of less than one.

An "indel" is a common form of polymorphism comprising a small insertion or deletion that is typically only a few nucleotides long.

A "computer-readable medium", is an information storage medium that can be accessed by a computer using a commercially available or custom-made interface. Exemplary computer-readable media include memory (e.g., RAM, ROM, flash memory, etc.), optical storage media (e.g., CD-ROM), magnetic storage media (e.g., computer hard drives, floppy disks, etc.), punch cards, or other commercially available media. Information may be transferred between a system of interest and a medium, between computers, or between computers and the computer-readable medium for storage or access of stored information. Such transmission can be electrical, or by other available methods, such as IR links, wireless connections, etc.

The word "subject" includes human, animal, avian, e.g., horse, donkey, pig, mouse, hamster, monkey, chicken, sheep, cattle, goat, buffalo.

Reference to "neoplasm" or "cancer" should be understood as a reference to a lesion, tumor or other encapsulated or unencapsulated mass or other form of growth which comprises neoplastic or cancer cells. A "cancer cell" should be understood as a reference to a cell exhibiting abnormal growth. The term "growth" should be understood in its broadest sense and includes reference to proliferation. In this regard, an example of abnormal cell growth is the uncontrolled proliferation of a cell. Another example is failed apoptosis in a cell, thus prolonging its usual life span. The neoplastic cell may be a benign cell or a malignant cell. In a certain embodiment, the subject neoplasm is a bladder tumor.

Reference to "DNA region" should be understood as a reference to a specific section of genomic DNA. These DNA regions are specified either by reference to a gene name or a set of chromosomal coordinates. Both the gene names and the chromosomal coordinates would be well known to, and understood by, the person of skill in the art. The chromosomal coordinates presented herein correspond to the Hg19 version of the genome. In general, a gene can be routinely identified by reference to its name, via which both its sequences and chromosomal location can be routinely obtained, or by reference to its chromosomal coordinates, via which both the gene name and its sequence can also be routinely obtained.

In reference to genes/DNA, the following should be noted as well. Reference to each of the genes/DNA regions detailed herein are understood as a reference to all forms of these molecules and to fragments or variants thereof. As would be appreciated by the person of skill in the art, some genes are known to exhibit allelic variation between individuals or single nucleotide polymorphisms. SNPs encompass insertions and deletions of varying size and simple sequence repeats, such as dinucleotide and trinucleotide repeats. Variants include nucleic acid sequences from the same region sharing at least 90%, 95%, 98%, 99% sequence identity i.e. having one or more deletions, additions, substitutions, inverted sequences etc. relative to the DNA regions described herein. Accordingly, certain present embodiments should be understood to extend to such variants which, in terms of the present diagnostic applications, achieve the same outcome despite the fact that minor genetic variations between the actual nucleic acid sequences may exist between individuals. The present embodiments should therefore be understood to extend to all forms of DNA which arise from any other mutation, polymorphic or allelic variation.

Cancer diagnosis as described herein refers to determining or classifying the nature of the cancer state, e.g., the mutational or genetic phenotype of a cancer or tumor, the clinical stage of a cancer associated with its progression, and/or the metastatic nature of the cancer. Cancer diagnosis based on genetic phenotyping can help guide proper therapeutic intervention as described herein.

Cancer prognosis as described herein includes determining the probable progression and course of the cancerous condition, and determining the chances of recovery and survival of a subject with the cancer, e.g., a favorable prognosis indicates an increased probability of recovery and/or survival for the cancer patient, while an unfavorable prognosis indicates a decreased probability of recovery and/or survival for the cancer patient. A subject's prognosis can be determined by the availability of a suitable treatment (i.e., a treatment that will increase the probability of recovery and survival of the subject with cancer). This aspect of certain present embodiments may further include selecting a suitable cancer therapeutic based on the determined prognosis and administering the selected therapeutic to the subject.

Prognosis also encompasses the metastatic potential of a cancer. For example, a favorable prognosis based on the presence or absence of a genetic phenotype can indicate that the cancer is a type of cancer having low metastatic potential, and the patient has an increased probability of long term recovery and/or survival. Alternatively, an unfavorable prognosis, based on the presence or absence of a genetic phenotype can indicate that the cancer is a type of cancer having a high metastatic potential, and the patient has a decreased probability of long term recovery and/or survival. Prognosis is in part assessed by pathologic grade and stage. Wherein grade is defined as papilloma, or low grade, or high grade based on standards set by the American Joint Committee on Cancer. Wherein stage is defined by the Tumor, Node, Metastasis (TNM) staging system. For example, tumor stage may be defined as T, T0, Ta, Tis, T1, T2, T2a, T2b, T3, T3a, T3b, T4a, T4b. For example, node stage may be defined as NX, N0, N1, N2, N3. For example, metastasis stage may be defined as M0, M1. In one embodiment, genomic phenotypes or combinations of one or more mutations or epigenetic alterations may be compared to a database containing genomic phenotypes and staging information and wherein this comparison approximates tumor stage and grade by computational measurement of urine genomic phenotypic similarity to other tumors with known stage, grade, and patients' outcomes in the database.

Another aspect of certain present embodiments is directed at identification of the type of bladder cancer present. Bladder cancer can be defined as transitional cell type or urothelial cancer, squamous cell bladder cancer, adenocarcinoma of the bladder, sarcoma of the bladder, small cell cancer of the bladder. In one aspect of the present embodiments, genomic phenotypes or combinations of one or more mutations or epigenetic alterations or one or more mutations or epigenetic alterations may be compared to a database containing genomic phenotypes and defining cancer cell type information and wherein this comparison approximates tumor cell type by computational measurement of urine genomic phenotypic similarity to other tumors with known cell type in the database. In another aspect of the present embodiments, genomic phenotypes or combinations of one or more epigenetic alterations may be used to generate in silico models approximating the tumor microenvironment and relative abundance of non-cancerous cells which may modulate the activity and biology of cancer cells.

Another aspect of certain present embodiments is directed to a method of monitoring cancer progression in a subject that involves obtaining first and second urine samples containing nucleic acid, at different points in time, from the subject having cancer. The nucleic acid in the samples is contacted with one or more reagents suitable for detecting the presence or absence of one or more mutations and/or epigenetic alterations in one or more genes associated with bladder cancer, and the presence or absence of the one or more mutations and/or epigenetic alterations in the one or more genes associated with bladder cancer is detected. The method further involves comparing the presence or absence of the one or more mutations and/or epigenetic alterations detected in the first urine sample nucleic acid to the presence or absence of the one or more mutations and/or epigenetic alterations detected in the second urine sample nucleic acid and monitoring cancer progression in the subject based on the comparison.

A change in the mutational and/or epigenetic alterations status of one or more genes associated with bladder cancer, for example, detecting the presence of a mutation and/or epigenetic alterations in the second urine sample whereas no mutation and/or epigenetic alteration was detected in the first urine sample, indicates that a change in the cancer phenotype has occurred with disease progression. This change may have therapeutic implications, i.e., it may signal the need to change the subject's course of treatment. The change can also be indicative of the progression of the cancer to a metastatic phenotype. Therefore, periodic monitoring of urine nucleic acid mutational and/or epigenetic status provides a means for detecting primary tumor progression, metastasis, and facilitating optimal targeted or personalized treatment of the cancerous condition.

The time between obtaining a first urine nucleic acid sample and a second, or any additional subsequent urine nucleic acid samples can be any desired period of time, for example, weeks, months, years, as determined is suitable by a physician and based on the characteristics of the primary tumor (tumor type, stage, location, etc.). In one embodiment of this aspect, the first sample is obtained before treatment and the second sample is obtained after treatment. Alternatively, both samples can be obtained after one or more treatments; the second sample obtained at some point in time later than the first sample. Alternatively, one or more samples can be obtained before presence of disease.

Mutations and/or epigenetic alterations in several genes have been shown to be associated with bladder cancer. Table 1 shows a list of genes from which to choose for assaying mutations and/or epigenetic alterations related to bladder cancer. Mutations can include insertions, deletions, duplications, amplifications, and translocations. Epigenetic features can include methylation of cytosine nucleotides. Other genes found to be associated with bladder cancer can also be used in a present embodiment based on empirical validation. Using individually synthesized DNA or RNA hybridization probes allows for modularity of hybrid capture libraries and iterative optimization (removal/addition of probes) based on empirical validation. Specificity of capture probes can be addressed computationally during the design of probes but also during sequencing validation. In a CLIA lab setting, an exemplary approach for validating inconclusive or unexpected results has been to complement hybrid capture with a secondary PCR amplicon based enrichment approach to provide coverage of regions not amenable to hybrid capture and to confirm novel results. Massively parallel amplification systems such as RainDance, AmpliSeq, and Wafergen provide high efficiency and uniformity for amplicon library preparation.

Any known methods for isolating cells from urine, of isolating cell-free nucleic acid in urine as well as nucleic acid from cells found in urine, are incorporated herein in their respective entireties. A urine preservation buffer may contain the following classes of reagents, microbial static agents such as EDTA, Isothiazolinone and/or its derivatives such as Methylisothiazolinone, antibiotics, pH Buffering reagents such as Tris salt, DNAse/RNAse inhibitors such as EDTA and Aurintricarboxylic acid, modifiers of nucleic acid hydration including chaotropic salts such as *Guanidinium* thiocyanate, Ammonium Acetate, Sodium Acetate, Sodium Dodecyl Sulfate. In one aspect, a urine preservation buffer results indicate preservation of DNA for at least 1 week at room temperature. Other buffers can be used per the knowledge and skill in the art. In one embodiment, the buffers and reagents are optimized to avoid co-precipitation of salts which inhibit many enzyme-based reactions such as PCR or ligation while simultaneously maximizing high yield from the sample.

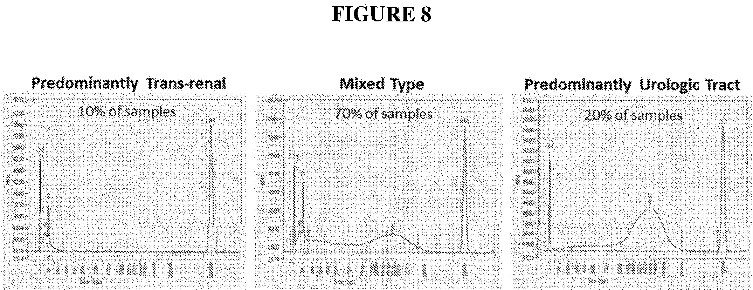

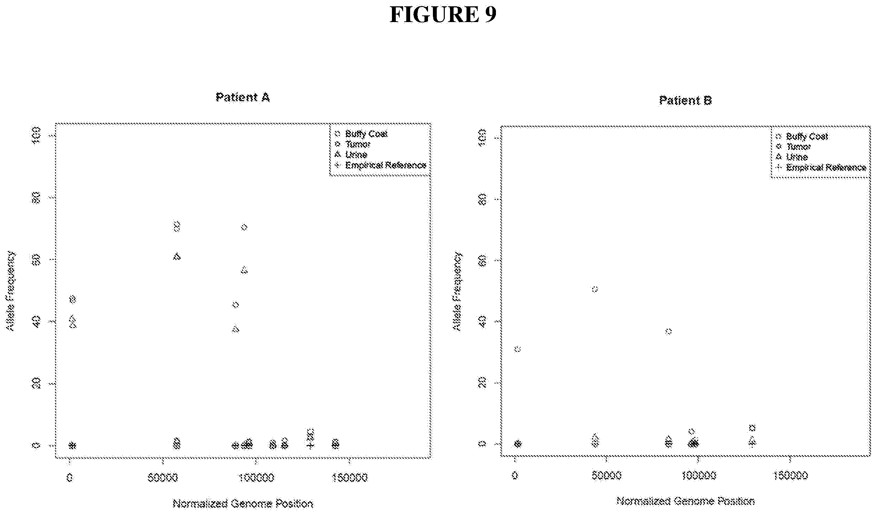

Cancer markers can be identified in both cell-associated and cell-free nucleic acids within urine. As shown in Figs. 8 and 9 the distribution of nucleic acid in urine as well as the relative abundance of cancer nucleic acid markers varies between these two populations and can be dependent on individual patient profiles. Due to patient variability, advantages exist in examining both of these nucleic acid populations together. As reflected in Fig. 8, the size distribution of these nucleic acids can also vary with cell-free nucleic acid often in the 50-200bp size range while cell-associated nucleic acid is often greater than 1,000bp. Some patients display wide range and variability of urine nucleic acid size while others contain primarily one fraction over the other. Variability in DNA fragmentation profiles is not only caused by collection and storage conditions, it is also a factor of the diverse physiology of the patients. In addition to time of day collected, certain individuals appear to have natural biases to one urine profile type over another. As reflected in Fig. 8, three types of patient profiles have been developed, "Predominantly Trans-renal" "Mixed type" and Predominantly Urologic Track. In individuals characterized as predominantly small/trans-renal profile, in order to obtain samples with improved nucleic acid profiles, it is also best to collect samples when urine incubation with the bladder has been maximized (early morning) and in other cases immediate voiding of urine into a preservation buffer which inhibits nuclease activity to prevent degradation of nucleic acid.

According to embodiments of the invention, theses respective patient profiles are determined using nucleic acid data, categorized and then compared to control groups of both heathy patients and previously determined patient profiles characterized by having bladder cancer.

Fig. 9 depicts dot plot graphs that represent the relationship between allele frequency in tumor and urine nucleic acid in patient matched samples where urine was collected while tumor was present in the bladder. The vertical axis is non-reference allele frequency and the horizontal axis is genomic position within targeted genomic region, the dots denote sample type and are described in the figure key. Patient A shows that some patients have high allele frequency concordance between tumor (range 42-71%) and urine (38-60%), where the majority of nucleic acid in urine is of tumor origin. Conversely, patient B shows another scenario where the abundance of tumor derived nucleic acid in urine is much lower and tumor (26-51%) and urine (0.3-2.2%) mutation abundance is discordant while urine mutations still maintain abundance above reference database ranges for those positions (grey X). Both Patient A and B demonstrate an additional characteristic of distinct allele frequency clusters within both tumor and urine samples. In one embodiment the extent or type of allele frequency clustering may be used as part of a diagnostic or prognostic disease algorithm. Accordingly, Fig. 8 demonstrates that different patients can manifest radically bladder cancer indications in urine and in significantly different manners, wherein some patients may provide numerous strong signals and others only infrequent detected markers. According to embodiments of the invention, the unique patient profile data is captured with respect to such patient profiles and is analyzed and then used in connection with subsequent sample collection, preparation and ongoing patient testing analysis. The subsequent detection, diagnosis and prognosis can therefore be catered to the individual patient profile and data from populations of similar patients that exhibit similar profiles can be collected and analyzed for subsequent treatment outcome analysis across wide populations.

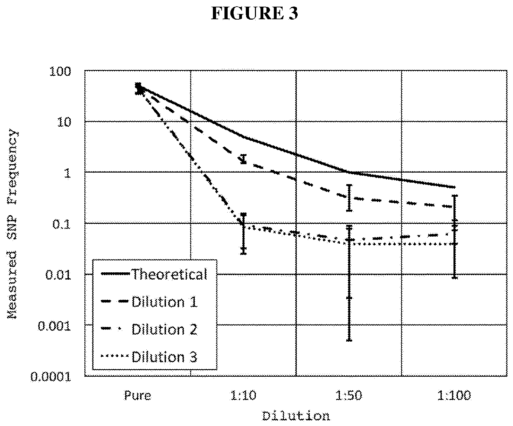

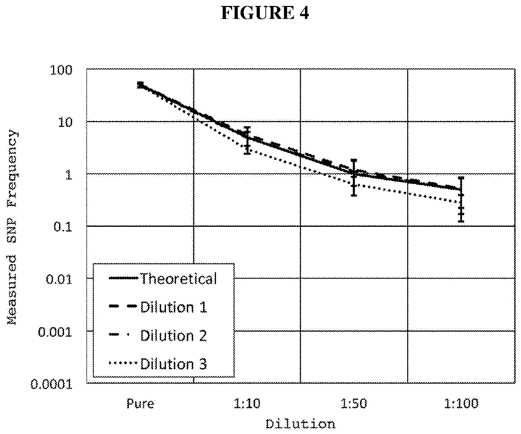

Now referring to Figs 3 and 4, graphs illustrating SNP frequency plotted against iterative dilution level of a reference sample are depicted with and without quality control metrics. The ability to detect cancer nucleic acid diluted in a background of normal nucleic acids in urine is dependent on library preparation efficiency which is in part modulated by a series of sample quality controls. To determine the impact of sample quality control on sequencing performance, reference samples with known variants were diluted into a background reference sample. Dilutions of DNA samples were performed as indicated (horizontal axis) by volumetric serial dilution, sequencing libraries were generated, samples sequenced and the allele frequency of known single nucleotide variants was calculated (vertical axis). Points on each line represent the mean performance of technical replicates, error bars are standard error of the mean. In two of three dilution series the measured allele frequency of single nucleotide variants was far below the theoretical expected dilution signal. After development of enhanced sample quality control methods, new dilution series were generated in which nucleic acid input was normalized using quality control results. These enhanced methods result in sequencing library preparation efficiency which match the expected dilution signal.

When library preparation efficiency is poor (poor ligation efficiency due to size of DNA, overloading of DNA, or presence of end-repair, A-tailing, and ligase enzyme inhibitors, or presence of single stranded DNA which is measured but cannot ligate) or when hybrid capture efficiency is poor (due to non-human nucleic acid) samples that perform like shown in Fig. 3 are expected, wherein variants in a sample are detected far below what would be expected based on amount of DNA that we thought we put in. In figure 4, after QCs are implemented to insure sufficient quantities of higher molecular weight DNA are input (capillary electrophoresis and real-time PCR), that the DNA that is of human origin (real time PCR, nitrates), that DNA is double stranded (capillary electrophoresis and fluorimetry), that the DNA is functional and of amplifiable quality (real-time PCR). When these are all taken together then our sequencing performance can look like in figure 4 where is performs as expected theoretically.

The nature and extent of Qualify Control features are in part depending on the nature of the sample. For example, Nitrates in urine indicates that there may be high bacterial levels. When bacterial DNA is abundant in nucleic acid extracted from urine it has the ability to disrupt the efficiency of hybrid capture. This disruption in hybrid capture is in part due to the fact that most nucleic acid quantification technologies do not distinguish between human and non-human DNA (UV absorbance, fluorimetry, and capillary electrophoresis all do not distinguish human from non-human nucleic acid). Efficient hybrid capture designed to enrich for human genes is dependent upon accurate up front DNA input into the reaction where this defined input is of human origin. Positive nitrate results can act as a flag in lab protocols and indicate that additional quality controls were necessary in which PCR is used to quantify the abundance of human DNA to non-human DNA so that sufficient human DNA can be loaded into the library preparation reaction. In some cases, the non-human DNA may be reach a level of abundance that despite human/non-human normalization, it begins to overload or actively inhibit library preparation (both the end-repair, A-tailing, ligation, or hybrid capture reactions). In this case steps are taken to actively destroy or deplete non-human sequences prior to library preparation (this may be performed by treatment with restriction enzymes targeting bacterial specific sequence motifs, differential nucleic acid methylation patterns e.g. methyl-CpG binding domains, described in http://dx.doi.org/10.1371/journal.pone.0076096, treatment with non-ionic surfactants such as saponin 0.025%, as described in http://jcm.asm.org/content/early/2016/01/07/JCM.03050-15).

As such, quality control measures for both the impact of non-human sequence on a library and enrichment efficiency and other subtle decreases in efficiency even when a sample was purported to be negative for urinary tract infection or was negative for nitrates by urine chemistry can improve assay performance. In this regard, even in "healthy" and "normal" urine samples bacterial and yeast levels from a normal microbiome can be sufficient to impact sequencing efficiency (See Figure 3 & 4). Further, traditional prior art biomarkers and definitions of urinary tract infection (nitrates, urine culture) are not as sensitive as our genomic approach which can result in discrepancies between early urinary tract infection testing and the level of non-human nucleic acid. For this reason, a more sensitive PCR based methodology may be implemented to distinguish human from non-human nucleic acid. In embodiments, the PCR reaction used is therefore designed against sequences that are specific to human, bacterial, yeast or viral nucleic acid sequences. Genes listed below are selected based on analysis of copy number variation data in bladder cancer to select for genomic regions which are copy number neutral. Alternatively, analyzing ALU elements can also avoid genomic copy number influencing the approximation of genome equivalents. In an embodiment, human specific PCR is performed in which the reaction primers are designed against ALU-element sequences and/or one of more of the genes selected from the following list: CTIF, MRO, STYX, TIMM9, PIGH, WRB, AIRE, MDFIC,PON3, ERMN, and RND3. PCR sequences are also selected that are in regions of the genome that do not vary with normal or cancer associated genomic copy number variation, such to allow more direct quantification of the number of genome equivalents present in a nucleic acid extraction.

Additional quality control steps relate to urine chemistry, including levels of pH, Hemaglobin, Myoglobin, Ketones, urobilinogen, and specific gravity. These markers are tested for and then used for normalization of mutation calling algorithms. These analytes may modify the chemical structure of nucleic acids in such ways to introduce errors in sequencing. One aspect of the empirical reference library (denoted in the algorithm flow diagram) is to use sequencing data from many samples with these abnormalities to build sequencing error pattern profiles for different analytical ranges of these analytes. These error models can then be used to then reduce sequencing errors and correct for potential false-positive signals within sequencing results.

Leukocyte esterase is a marker for white blood cells (WBCs) in urine. In urine samples with high levels of white blood cells a tumor signature may be diluted by the normal DNA present in these cells. Embodiments of the invention involve two approaches to correct for high WBCs, (1) active depletion prior to urine extraction (examples of methods including separation through differential centrifugation or exposure to solute gradients, differential lysis through treatment with salt solutions, use of cell surface markers to deplete by antibody pull down or column), and/or (2) the adjustment of the algorithm thresholds to account for elevated levels on non-cancer DNA.

Specific Gravity and creatinine values can serve as surrogates for kidney function and urine dilution. In some cases, these markers can approximate the levels of systemic (trans-renal) nucleic acid relative to urologic tract nucleic acid. These markers may also inform how size distributions correlate to systemic vs. urologic tract nucleic acid. In embodiments the values are tested, a reference library created and the algorithm can be appropriately adjusted. Specific gravity and pH. values may correlate to the levels of double stranded DNA vs. single stranded DNA present in a urine sample.

In an embodiment, a method of total nucleic acid processing and extraction from urine comprises:
(i) a step of incubation of urine in a lysis solution. Such a solution can optionally contain a detergent, a salt, e.g. 5M NaCl, chaotropic salts (e.g. Guanidinium thiocyanate, Sodium Acetate), protein digesting enzymes such as Protinase K, and isopropyl alcohol, or ethanol;
(ii) a step of addition of a nucleic acid binding substrate, such as a silica resin slurry (Norgen Urine DNA kit), or magnetic negatively charged nucleic acid binding beads (such as Invitrogen MagMax total nucleic acid kit) or a siliconized column (such as Qiagen QIAprep Spin Miniprep Kit);
(iii) a step of washing of the bound DNA with lysis solution;
(iv) a step of elution of the DNA in a buffered solution, e.g. containing Tris and EDTA; and
(v) an optional step of conversion and tagging/barcoding of RNA into cDNA.

This final optional step can be done by any method known in the art. For example, using ClonTech's Smarter (Switching Mechanism at 5' End of RNA Template) cDNA conversion kit. This technology allows the efficient incorporation of known sequences at both ends of cDNA during first strand synthesis, without adaptor ligation. The presence of these known sequences is crucial for downstream applications where DNA, and RNA derived cDNA (generated by the SMARTER kit), are prepared in the same library and sequenced together within a single sequencing run. Inclusion of both DNA and RNA within a single library permits genomic translocations to be identified from the RNA/cDNA while mutations and epigenetic alterations can be identified from DNA or RNA. SMARTER incorporated known sequences allow downstream informatic deconvolution of DNA and RNA unique signals.

In one embodiment, the extracted nucleic acid is DNA. In another embodiment, the extracted nucleic acid is RNA. RNAs are in certain embodiments reverse-transcribed into complementary DNAs. Such reverse transcription may be performed alone or in combination with an amplification step, e.g., using reverse transcription polymerase chain reaction (RT-PCR), which may be further modified to be quantitative, e.g., quantitative RT-PCR as described in U.S. Pat. No. 5,639,606, which is hereby incorporated by reference in its entirety.

In one embodiment, the extracted nucleic acids, including DNA and/or RNA, are analyzed directly without an amplification step. Direct analysis may be performed with different methods including, but not limited to, nanostring technology. NanoString technology enables identification and quantification of individual target molecules in a biological sample by attaching a color coded fluorescent reporter to each target molecule. This approach is similar to the concept of measuring inventory by scanning barcodes. Reporters can be made with hundreds or even thousands of different codes allowing for highly multiplexed analysis. The technology is described in a publication by Geiss et al. "Direct Multiplexed Measurement of Gene Expression with Color-Coded Probe Pairs," Nat Biotechnol 26(3): 317-25 (2008), which is hereby incorporated by reference in its entirety.

In another embodiment, it may be beneficial or otherwise desirable to amplify the nucleic acid for enrichment of known bladder cancer genes prior to analyzing it. Methods of nucleic acid amplification are commonly used and generally known in the art. If desired, the amplification can be performed such that it is quantitative. Quantitative amplification will allow quantitative determination of relative amounts of the various nucleic acids. Enrichment of bladder cancer genes can occur by PCR, emulsion PCR, massively multiplexed PCR, allele specific PCR, Molecular inversion probes, fragmentation and binding of site specific probes followed by circularization, or hybrid capture. A certain embodiment uses hybrid capture in which adapter ligated DNA libraries are incubated with 1. an oligo nucleotide complementary to adapter sequence (blocking oligo) 2. A buffer optimized for DNA hybridization (Illumina Nextera) and 3. A set of biotinylated custom synthesized oligo nucleotides complementary to genomic regions of interest (Nextera Custom Capture, or IDT XGen lockdown probes).

Nucleic acid amplification methods include, without limitation, polymerase chain reaction (PCR) (U.S. Pat. No. 5,219,727, which is hereby incorporated by reference in its entirety) and its variants such as in situ polymerase chain reaction (U.S. Pat. No. 5,538,871, which is hereby incorporated by reference in its entirety), quantitative polymerase chain reaction (U.S. Pat. No. 5,219,727, which is hereby incorporated by reference in its entirety), nested polymerase chain reaction (U.S. Pat. No. 5,556,773), self-sustained sequence replication and its variants (Guatelli et al. "Isothermal, In vitro Amplification of Nucleic Acids by a Multienzyme Reaction Modeled after Retroviral Replication," Proc Natl Acad Sci USA 87(5): 1874-8 (1990), which is hereby incorporated by reference in its entirety), transcriptional amplification system and its variants (Kwoh et al. "Transcription-based Amplification System and Detection of Amplified Human Immunodeficiency Virus type 1 with a Bead-Based Sandwich Hybridization Format," Proc Natl Acad Sci USA 86(4): 1173-7 (1989), which is hereby incorporated by reference in its entirety), Qb Replicase and its variants (Miele et al. "Autocatalytic Replication of a Recombinant RNA." J Mol Biol 171(3): 281-95 (1983), which is hereby incorporated by reference in its entirety), cold-PCR (Li et al. "Replacing PCR with COLD-PCR Enriches Variant DNA Sequences and Redefines the Sensitivity of Genetic Testing." Nat Med 14(5): 579-84 (2008), which is hereby incorporated by reference in its entirety) or any other nucleic acid amplification methods, followed by the detection of the amplified molecules using techniques known to those of skill in the art. Especially useful are those detection schemes designed for the detection of nucleic acid molecules if such molecules are present in very low numbers.

Detecting the presence or absence of one or more mutations and/or epigenetic alterations in bladder cancer genes in a tumor or urine-derived nucleic acid sample from a subject can be carried out using methods that are well known in the art.

In one embodiment, the one or more mutations in the one or more identified genes is detected using a hybridization assay. In a hybridization assay, the presence or absence of a gene mutation is determined based on the hybridization of one or more allele-specific oligonucleotide probes to one or more nucleic acid molecules in the DNA sample from the subject. The oligonucleotide probe or probes comprise a nucleotide sequence that is complementary to at least the region of the gene that contains the mutation of interest. The oligonucleotide probes are designed to be complementary to the wildtype, non-mutant nucleotide sequence and/or the mutant nucleotide sequence of the one or more genes to effectuate the detection of the presence or the absence of the mutation in the sample from the subject upon contacting the sample with the oligonucleotide probes. A variety of hybridization assays that are known in the art are suitable for use in the methods of the present embodiments. These methods include, without limitation, direct hybridization assays, such as northern blot or Southern blot (see e.g., Ausabel et al., Current Protocols in Molecular Biology, John Wiley & Sons, NY (1991)).

Alternatively, direct hybridization can be carried out using an array based method where a series of oligonucleotide probes designed to be complementary to a particular non-mutant or mutant gene region are affixed to a solid support (glass, silicon, nylon membranes). A labeled DNA or cDNA sample from the subject is contacted with the array containing the oligonucleotide probes, and hybridization of nucleic acid molecules from the sample to their complementary oligonucleotide probes on the array surface is detected. Examples of direct hybridization array platforms include, without limitation, the Affymetrix GeneChip or SNP arrays and Illumina's Bead Array.

In another embodiment, a sample is bound to a solid support (often DNA or PCR amplified DNA) and labeled with oligonucleotides in solution (either allele specific or short so as to allow sequencing by hybridization).

Detecting specific mutations can be accomplished by methods known in the art for detecting sequences at specific sites. For example, fluorescence-based techniques (Chen, X. et al., Genome Res. 9(5): 492-98 (1999)), utilizing PCR, LCR, Nested PCR and other techniques for nucleic acid amplification. Specific commercial methodologies available include, but are not limited to, TaqMan genotyping assays and SNPlex platforms (Applied Biosystems), gel electrophoresis (Applied Biosystems), mass spectrometry (e.g., MassARRAY system from Sequenom), minisequencing methods, real-time PCR, Bio-Plex system (BioRad), CEQ and SNPstream systems (Beckman), array hybridization technology (e.g., Affymetrix GeneChip; Perlegen), BeadArray Technologies (e.g., Illumina GoldenGate and Infinium assays), array tag technology (e.g., Parallele), and endonuclease-based fluorescence hybridization technology (Invader; Third Wave). Some of the available array platforms, including Affymetrix SNP Array 6.0 and Illumina CNV370-Duo and 1M BeadChips, include SNPs that tag certain CNVs. This allows detection of copy number variations (CNVs) via surrogate SNPs included in these platforms. Thus, by use of these or other methods available to the person skilled in the art, one or more mutations and/or epigenetic alterations can be identified.

In certain embodiments, a mutation in a gene is detected by sequencing technologies. Obtaining sequence information about an individual identifies particular nucleotides in the context of a sequence. For SNPs, sequence information about a single unique sequence site is sufficient to identify alleles at that particular SNP. For markers comprising more than one nucleotide, sequence information about the nucleotides of the individual that contain the polymorphic site identifies the alleles of the individual for the particular site. The sequence information can be obtained from a nucleic acid sample from the urine of the subject or individual.

Various methods for obtaining nucleic acid sequence are known to the skilled person, and all such methods are useful for practicing the embodiments. Sanger sequencing is a well-known method for generating nucleic acid sequence information. Recent methods for obtaining large amounts of sequence data have been developed, and such methods are also contemplated to be useful for obtaining sequence information. These include pyrosequencing technology (Ronaghi, M. et al. Anal Biochem 267:65-71 (1999); Ronaghi, et al., Biotechniques 25:876-878 (1998)), e.g. 454 pyrosequencing (Nyren, P., et al. Anal Biochem 208:171-175 (1993)), Illumina/Solexa sequencing technology (www.illumina.com; see also Strausberg, R L, et al. Drug Disc Today 13:569-577 (2008)), and Supported Oligonucleotide Ligation and Detection Platform (SOLiD) technology (Applied Biosystems, www.appliedbiosystems.com); Strausberg, R L, et al. Drug Disc Today 13:569-577 (2008). The foregoing are incorporated by reference in their respective entireties.

Other common genotyping methods include, but are not limited to, restriction fragment length polymorphism assays; amplification based assays such as molecular beacon assays, nucleic acid arrays, high resolution melting curve analysis (Reed and Wittwer, "Sensitivity and Specificity of Single-Nucleotide Polymorphism Scanning by High Resolution Melting Analysis," Clinical Chem 50(10): 1748-54 (2004), which is hereby incorporated by reference in its entirety); allele-specific PCR (Gaudet et al., "Allele-Specific PCR in SNP Genotyping," Methods Mol Biol 578: 415-24 (2009), which is hereby incorporated by reference in its entirety); primer extension assays, such as allele-specific primer extension (e.g., Illumina^{™} Infinium^{™} assay), arrayed primer extension (see Krjutskov et al., "Development of a Single Tube 640-plex Genotyping Method for Detection of Nucleic Acid Variations on Microarrays," Nucleic Acids Res. 36(12) e75 (2008), which is hereby incorporated by reference in its entirety), homogeneous primer extension assays, primer extension with detection by mass spectrometry (e.g., Sequenom^{™} iPT EX SNP genotyping assay) (see Zheng et al., "Cumulative Association of Five Genetic Variants with Prostate Cancer," N. Eng. J. Med. 358(9):910-919 (2008), which is hereby incorporated by reference in its entirety), multiplex primer extension sorted on genetic arrays; flap endonuclease assays (e.g., the Invader^{™} assay) (see Olivier M., "The Invader Assay for SNP Genotyping," Mutat. Res. 573 (1-2) 103-10 (2005), which is hereby incorporated by reference in its entirety); 5' nuclease assays, such as the TaqMan^{™} assay (see U.S. Pat. No. 5,210,015 to Gelfand et al. and U.S. Pat. No. 5,538,848 to Livak et al., which are hereby incorporated by reference in their entirety); and oligonucleotide ligation assays, such as ligation with rolling circle amplification, homogeneous ligation, OLA (see U.S. Pat. No. 4,988,617 to Landgren et al., which is hereby incorporated by reference in its entirety), multiplex ligation reactions followed by PCR, wherein zipcodes are incorporated into ligation reaction probes, and amplified PCR products are determined by electrophoretic or universal zipcode array readout (see U.S. Pat. Nos. 7,429,453 and 7,312,039 to Barany et al., which are hereby incorporated by reference in their entirety). Such methods may be used in combination with detection mechanisms such as, for example, luminescence or chemiluminescence detection, fluorescence detection, time-resolved fluorescence detection, fluorescence resonance energy transfer, fluorescence polarization, mass spectrometry, and electrical detection. In general, the methods for analyzing genetic aberrations are reported in numerous publications, not limited to those cited herein, and are available to those skilled in the art. The appropriate method of analysis will depend upon the specific goals of the analysis, the condition/history of the patient, and the specific cancer(s), diseases or other medical conditions to be detected, monitored or treated.

Alternatively, the presence or absence of one or more mutations identified *supra* can be detected by direct sequencing of the genes, or in one embodiment particular gene regions comprising the one or more identified mutations, from the patient sample. Direct sequencing assays typically involve isolating DNA sample from the subject using any suitable method known in the art, and cloning the region of interest to be sequenced into a suitable vector for amplification by growth in a host cell (e.g. bacteria) or direct amplification by PCR or other amplification assay. Following amplification, the DNA can be sequenced using any suitable method. As certain sequencing methods involve high-throughput next generation sequencing (NGS) to identify genetic variation. Various NGS sequencing chemistries are available and suitable for use in carrying out the embodiments, including pyrosequencing (Roche^{™} 454), sequencing by reversible dye terminators (Illumina^{™} HiSeq, Genome Analyzer and MiSeq systems), sequencing by sequential ligation of oligonucleotide probes (Life Technologies^{™} SOLiD), and hydrogen ion semiconductor sequencing (Life Technologies^{™}, Ion Torrent^{™}). Alternatively, classic sequencing methods, such as the Sanger chain termination method or Maxam-Gilbert sequencing, which are well known to those of skill in the art, can be used to carry out the methods of the present embodiments.

Certain present embodiments also provide kits which are useful for carrying out the disclosures set forth herein. The present kits comprise one or more container means containing the above-described assay components. The kit also comprises other container means containing solutions necessary or convenient for carrying out the embodiments. The container means can be made of glass, plastic or foil and can be a vial, bottle, pouch, tube, bag, etc. The kit may also contain written information, such as procedures for carrying out certain present embodiments or analytical information, such as the amount of reagent contained in the first container means. The container means may be in another container means, e.g. a box or a bag, along with the written information.

The following examples are included to demonstrate certain embodiments hereof. It should be appreciated by those of skill in the art that the techniques disclosed in the examples which follow represent techniques discovered by the inventors and thought to function well in the practice of the embodiments, and thus can be □considered to constitute certain modes for its practice. However, those of skill in the art should, in light of the present disclosure, □appreciate that many changes can be made in the specific embodiments which are disclosed and still obtain a like or similar result without departing from the spirit and scope of what is described. □

All documents cited herein are hereby □incorporated in their entirety by reference thereto.

The following materials and methods were used in the Examples below.

### Example 1

### DNA Repair and Sequencing Adapter Ligation

1. Repair of DNA strand nicks or gaps by treatment with one or more of the following enzymes: *Tag* DNA Ligase, Endonuclease IV, *Bst* DNA Polymerase, Fpg, Uracil-DNA Glycosylase (UDG), T4 PDG (T4 Endonuclease V) and Endonuclease VIII, polynucleotide kinase, mammalian DNA polymerase β and/or DNA ligase I
2. Repair and A-tailing of DNA ends by treatment of DNA with one or more of the following enxymes: T4 DNA Polymerase and Klenow Fragment
3. T4-ligation of a sequencing adapter and nucleic acid insert where the adapter is an Illumina TruSeq style adapter or equivalent. In an embodiment the adapter contains an 8-base pair sample barcode in the double stranded stem portion of adapter and the same barcode is present on both the p5 and p7 ends. In such embodiments matched dual index barcodes are used to avoid low frequency adapter contamination or adapter swaping/jumping between pooled samples. The adapter may also contain a diverse library of defined or random sequences in either the stem or y-portion of the adapter. And in which these defined or random sequences are used in part to tag an individual molecule prior to library amplification.
4. Or alternatively in place of steps 2 & 3: nucleic acid inserts are consecutively ligated to single strand adapter molecules as described in Nature Protocols 8, 737-748 (2013). Briefly, DNA is treated with a phosphatase to remove residual phosphate groups from the 5' and 3' ends of the DNA strands. A 5'-phosphorylated adapter oligonucleotide, and a long 3'-biotinylated spacer arm, is ligated to the 3'ends of the DNA strands using CircLigase II. The adapter-ligated molecules, as well as excess adapter molecules, are immobilized on streptavidin beads, and a primer complementary to the adapter is used to copy the template strand. This reaction is performed using Bst polymerase 2.0. After removal of 3' overhangs using T4 DNA polymerase, a second adapter is joined to the newly synthesized strands by blunt-end ligation with T4 DNA ligase. To prevent ligation between adapters, only one adapter strand is ligatable, whereas the other is blocked by a 3'-terminal dideoxy modification. After washing away excess adapter, the library molecules are released from the beads by heat denaturation
5. Design of the adapter sequences (used in steps 3 or 4) to include a specific number of DNA bases positioned within the adapter sequence (between 6-10 nucleotides in length) which are a degenerate or random sequence or in which the 6-10 nucleotide sequence is one of many (50-200 unique) defined sequences. And in which these adapters with divergently defined or degenerate sequences are present within the same mixture so as to create a diverse library of unique adapter sequences. And in which these unique sequences are subsequently used (in combination with other variables such as DNA insert start and stop site) to uniquely identify the clonal origin of an insert molecule following PCR amplification of a diverse population of adapter ligated insert molecules.

### Example 2

### (Enrichment of Known bladder cancer genes)

Enrichment of bladder cancer genes can occur by PCR, emulsion PCR, massively multiplexed PCR, allele specific PCR, Molecular inversion probes, fragmentation and binding of site specific probes followed by circularization, or hybrid capture.

An embodiment uses hybrid capture in which adapter ligated DNA libraries are incubated with 1. An oligo nucleotide complementary to adapter sequence (blocking oligo) 2. A buffer optimized for DNA hybridization (Illumina Nextera) and 3. A set of biotinylated custom synthesized oligo nucleotides complementary to genomic regions of interest (Nextera Custom Capture, or IDT XGen lockdown probes).

A series of incubations at various temperatures to promote hybridization of oligos to their target sequences.

Incubation of the hybridization reaction with strepavadin beads to enrich bound oligos from the solution. Washing and elution of the bound oligos from the beads.

A second repeated hybrid capture reaction with enriched fraction and custom oligos to further enrich for targets of interest.

Capture of bound oligos with strepavadin beads, wash and elution from the beads.

Load enriched sample onto sequencing machine.

### Example 3

### (Data Analysis Methods and Utilization and Interpretation of Results)

1. Deconvolution of DNA and cDNA sequences based on known sequences.
2. Mapping of DNA and cDNA reads to a reference genome.
3. Identification of molecular clonal families using unique pairs of degenerate or defined adapter sequences (both on the 5-prime and 3-prime ends of the molecule) and start/stop sites of DNA inserts.
4. Within clonal families, comparison of sequencing reads for base-pair call discrepancies.
5. Filtering or correction of discrepancies within an individual clone through a voting process in which the predominant base call at a particular location wins and is defined as the true base call and those base calls not present in a majority of molecules from the same clonal origin loose and are replaced with the predominant base call within that individual family.
6. Counting of the number of unique molecular/clonal families identified for a particular gene and comparing these counts to a set of reference genes within the same sample and also comparing these counts to an empirical distribution of counts for that gene across multiple samples. Copy number loss or copy number gains are identified when unique counts for a gene vary above a defined threshold relative to reference genes and/or empirical distributions.
7. Analysis of cDNA sequences for translocations or fusions of specific genes by reading through the break site on a sequence read.
8. Comparison of mutations and copy number counts between DNA and cDNA for confirmation of called mutational events.
9. Utilization of quantitative abundance of urine based mutational, and/or copy number changes, and/or translocations to determine the presence or absence of bladder cancer in a patient previously treated for bladder cancer.
10. Utilization of quantitative abundance of urine based mutational, and/or copy number changes, and/or translocations to determine the prognosis or risk of disease progression in a patient diagnosed with bladder cancer.
11. Utilization of quantitative abundance of urine based mutational, and/or copy number changes, and/or translocations to diagnosis bladder cancer in patients presenting with blood in their urine.
12. Utilization of quantitative abundance of urine based mutational, and/or copy number changes, and/or translocations to screen for bladder cancer or other cancers risk in asymptomatic or otherwise believed to be healthy individuals and/or high risk populations such as cigarette smokers, individuals with histories of occupational carcinogen exposures, individuals with histories of drinking water from wells or ground water contaminated with arsenic or other suspected carcinogens, or individuals living within geographical cancer hotspots.
13. Utilization of quantitative abundance of urine based mutational, and/or copy number changes, and/or translocations to perform short term individual screening for genotoxic stress induced by an external stimulus (testing in the hours to days to weeks following exposures) such as assessing potential genotoxicity when testing a new pharmaceutical product in mammals, or stratifying an individual's cancer risk from exposures to environmental or recreational carcinogens such as smog or products of combustion, alcohol, tobacco, UV radiation. Changes in mutational burden may be transient or persistent, and these genomic changes may be tracked longitudinally over time.

### Example 4

DNA is abundant in urine and can be optimally extracted for measurement of bladder cancer genomic biomarkers

In order to improve upon previous attempts to minimally detect bladder cancer in urine, embodiments focus on urine DNA as an analyte because of technical advances in next generation DNA sequencing that permit massively multiplexed analysis of tens to thousands of genes in a single sequencing reaction. DNA also has the advantage of being relatively stable and undergoes unique changes during tumor formation that are highly specific to cancer.

To assess the viability of utilizing urine DNA, DNA extraction from 20-100ml of urine is performed and optimized, using multiple extraction approaches. Total DNA yield is measured using a fluorescent double strand DNA binding dye assay (QuantIt, Life Technologies), capillary electrophoresis, and Real-Time PCR.PCR amplification efficiency was measured using quantitative real- time PCR amplification of the RNaseP gene from multiple urine samples. Subsequent analysis demonstrates superior yield and enhanced PCR amplification (lower threshold cycle (Ct)) when DNA is extracted using a functionalized magnetic bead approach. In embodiments positively charged functionalized magnetic beads provide advantageous extraction yields when used in low volume, low concentration, or degraded samples.

### Example 5

To further validate the types of urine DNA as effective disease biomarkers, cell pellet associated and urine cell free DNA is analyzed. Wherein these two populations, and various size fractionations thereof, are compared to each other to determine where the most abundance disease signals exist as defined by a prior analysis of matched tumor tissue. Further where the differences in disease marker abundance within these populations is compared to urine chemistry, urine cytology, nucleic acid fragmentation patterns, and clinical correlates and wherein these correlations are used to develop algorithms that predict for future patients which nucleic acid population will contain the most abundant level of disease specific biomarkers.

### Example 6

### (Development of a biomarker panel which encompasses the genomic diversity of bladder cancer)

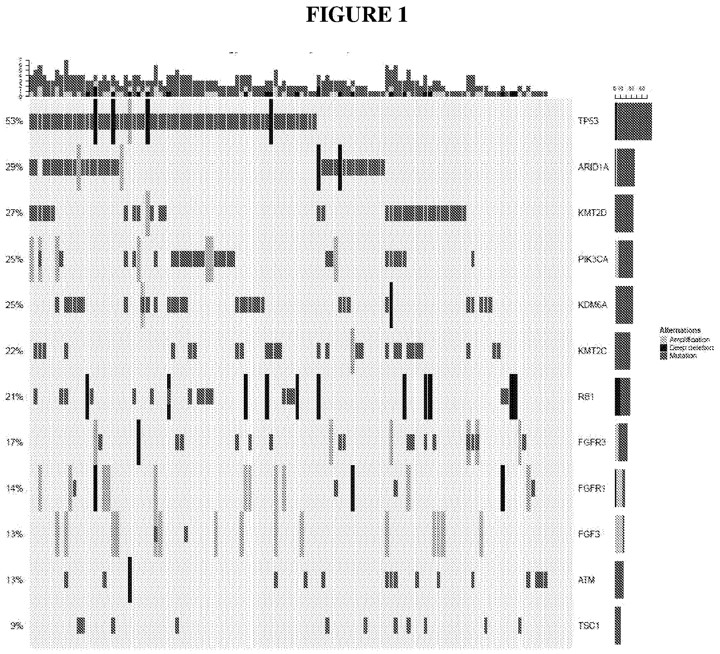

Significant developments in nucleic acid sequencing capacity, speed, sensitivity, and declines in cost have led to rapid adoption of cancer DNA sequencing in clinical molecular pathology labs. One significant shortcoming in previous FDA approved assays to monitor bladder cancer is that the biomarkers used have not been specific (detecting hematuria or inflammation) or they do not fully encompass the proteomic or genomic diversity of the disease. In order to improve upon prior art bladder cancer tests, an in particular their low sensitivity, specific embodiments of the present inventions are directed to a panel of multiple DNA bladder cancer biomarkers which better encompass the genomic diversity of bladder cancer. In order to assess the efficacy of using NGS for monitoring bladder cancer mutational burden, a panel of multiplexed amplicon based library enrichment reagents that focus on 12 recurrently mutated or amplified genes in bladder cancer (Figure 1) have been developed. In this Fig. 1 the gene panel is represented as a matrix, each row a unique gene in the panel and each column a unique patient in the bladder cancer TCGA dataset. Within the main matrix, columns represent unique patients, row genes. Cells are coded with the type of variant associated within a gene for a particular patient and gene, cell coding is denoted in the alterations legend (right).

Plot inlayed to right of matrix represents the abundance and type of mutation variants associated with a particular gene across this population. The top inlayed bar graph above the matrix represents the number and type of unique events on a per patient basis. Based on this analysis, 127 patients (94.8%) contain one or more abnormality in our biomarker panel with an average of 2.2 SNVs per patient. This panel was developed to create a minimally informative DNA based disease signature that encompasses the genomic diversity of the disease but also allows economical high depth sequencing, enrichment of fragmented DNA, and multiplexed sample analysis in a single sequencing run. Our preliminary embodiment of the panel amplifies 68 kb of genomic material using 690 PCR amplicons, provides 93% coverage of the target genes and very high (>99%) predicted on-target gene enrichment by blast alignment.

### Example 7

### (Sensitive and specific detection of bladder cancer burden)

To validate our assay disclosed herein , we analyze 11 control bladder cancer cell lines which have been previously sequenced by whole exome sequencing ((J. Barretina, G. Caponigro, N. Stransky, K. Venkatesan, A. A. Margolin, S. Kim, C. J. Wilson, J. Lehár, G. V. Kryukov, D. Sonkin, A. Reddy, M. Liu, L. Murray, M. F. Berger, J. E. Monahan, P. Morais, J. Meltzer, A. Korejwa, J. Jané-Valbuena, F. A. Mapa, J. Thibault, E. Bric-Furlong, P. Raman, A. Shipway, I. H. Engels, J. Cheng, G. K. Yu, J. Yu, P. Aspesi, M. de Silva, K. Jagtap, M. D. Jones, L. Wang, C. Hatton, E. Palescandolo, S. Gupta, S. Mahan, C. Sougnez, R. C. Onofrio, T. Liefeld, L. MacConaill, W. Winckler, M. Reich, N. Li, J. P. Mesirov, S. B. Gabriel, G. Getz, K. Ardlie, V. Chan, V. E. Myer, B. L. Weber, J. Porter, M. Warmuth, P. Finan, J. L. Harris, M. Meyerson, T. R. Golub, M. P. Morrissey, W. R. Sellers, R. Schlegel, and L. A. Garraway, "The Cancer Cell Line Encyclopedia enables predictive modelling of anticancer drug sensitivity," Nature, vol. 483, no. 7391, pp. 603-607, Mar. 2012); and (S. A. Forbes, D. Beare, P. Gunasekaran, K. Leung, N. Bindal, H. Boutselakis, M. Ding, S. Bamford, C. Cole, S. Ward, C. Y. Kok, M. Jia, T. De, J. W. Teague, M. R. Stratton, U. McDermott, and P. J. Campbell, "COSMIC: exploring the world's knowledge of somatic mutations in human cancer," Nucleic Acids Res., p. gku1075, Oct. 2014)). We chose these cell lines for their dynamic range within our panel, some lines containing no mutations and other lines contain multiple mutations. This analysis allows us to identify and mask out recurrent false-positive calls due to recurrent mapping errors or due to redundant (homopolymer) sequence context. Sensitivity of our pipeline is optimized by establishing multiple sequencing quality thresholds for alignment, base call and mutation call quality scores, loci specific read depth, and variant allele frequencies.

Using this refined mutation calling pipeline, an analysis on 14 cancer patients with diverse tumor stage, grade and clinical subtype (analysis of blood, tumor, and pre-surgery urines) was performed. Expansion of the panel to include additional genomic regions frequently mutated in bladder carcinoma *in situ* and other clinical subtypes of bladder cancer is supported. Expansion of the panel is conceived to further benefit assay sensitivity as performance increases with increasing numbers of mutations that can be monitored in a patient.

To assess the specificity of this type of approach, embodiments of the invention have validated the panel on 7 non-cancer controls (blood and urine). This cohort included patients with diverse urologic conditions including benign prostate hyperplasia, urinary retention, kidney stones, an individual seeking fertility consult and health controls. Among these, 2 patients were cigarette smokers with 10 & 60 pack years of smoking history. Future studies will expand the non-cancer control cohort to include further analysis of smokers and individuals with chronic urologic inflammatory disease as some of these patients may contain panel mutations in the absence of clinically detectible bladder cancer.

### Example 8

### (Longitudinal analysis of urine DNA can predict future disease recurrence)

To assess the ability of this approach to predict longitudinal disease recurrence, a further embodiment of the invention involves the analysis of two patients with known recurrence and long term longitudinal follow up including urine samples collected between trans-urethral resections of primary and recurrent tumors.

Using PCR amplicon based library enrichment, a lower limit of allele detection ranging from ~1-5% allele fraction depending on sequencing depth and amplicon performance was determined. An analysis pipeline was iteratively improved with increased data collection, including the recalibration of base quality scores, application of thresholds and modification of mutation calling algorithms to filter out recurrent panel specific mapping errors and analytical noise.

### Example 9

### (Design of an enhanced genomic panel which encompasses the diversity of bladder cancer)

Adoption of hybrid capture based library enrichment methodologies, deeper sequencing, and interrogation of a more diverse and encompassing set of biomarkers has the ability in an exemplary embodiment to enhance the sensitivity of the UriSeq recurrence assay by up to 2 orders of magnitude. We chose to focus exclusively on mutations (single nucleotide variants) as opposed to SNV and copy number alterations. Current algorithms for detection of SNVs are more sensitive at lower sequencing coverage than algorithms for detection of copy number variation and provide a good compromise between sensitivity and sequencing cost. To expand the panel of biomarkers assessed, we established a set of ranking criteria to prioritize recurrently mutated genes for inclusion in an enhanced panel. These criteria include: 1. Prevalence of recurrent mutations. 2. Prioritization of known oncogenes. 3. The size of the gene and its marginal cost of analysis (accounting for limitations in the number of probes which can be pooled into a single reaction). 4. Mutual exclusivity of mutations and the number of unique patients captured by addition of a gene or exon to the panel. 5. Differential prevalence of a mutated gene in unique clinical subtypes (e.g. enrichment in CIS, low grade or high grade lesions).

Based on these criteria, an embodiment directed to an enhanced panel targeting 750 exons in 23 genes for inclusion in the recurrence assay is provided. The comprehensive nature of this revised gene panel was validated computationally using the COSMIC database and 2 other publically available bladder cancer data sets, summarized in Table 2 ((The Cancer Genome Atlas Research Network, "Comprehensive molecular characterization of urothelial bladder carcinoma," Nature, vol. 507, no. 7492, pp. 315-322, Mar. 2014); (S. A. Forbes, D. Beare, P. Gunasekaran, K. Leung, N. Bindal, H. Boutselakis, M. Ding, S. Bamford, C. Cole, S. Ward, C. Y. Kok, M. Jia, T. De, J. W. Teague, M. R. Stratton, U. McDermott, and P. J. Campbell, "COSMIC: exploring the world's knowledge of somatic mutations in human cancer," Nucleic Acids Res., p. gku1075, Oct. 2014); and (P. H. Kim, E. K. Cha, J. P. Sfakianos, G. Iyer, E. C. Zabor, S. N. Scott, I. Ostrovnaya, R. Ramirez, A. Sun, R. Shah, A. M. Yee, V. E. Reuter, D. F. Bajorin, J. E. Rosenberg, N. Schultz, M. F. Berger, H. A. Al-Ahmadie, D. B. Solit, and B. H. Bochner, "Genomic Predictors of Survival in Patients with High-grade Urothelial Carcinoma of the Bladder," Eur. Urol., Aug. 2014)).

This design increases the percent of patients covered by the assay and increases the average number of SNVs per patient.

| Table 2: Summary of studies used for design a *silico* validation of an enhanced gene panel | | | |
|---|---|---|---|
| **Study** | **Study size (# patients)** | **Patients encompassed (%)** | **Average # events per patient** |
| Kim PH, et al. 2014 | 109 | 98 | 3.5 |
| TCGA, 2014 | 134 | 96 | 3.3 |

*In silico* validation based on these previous studies may underestimate the percent of patients that will be encompassed by this biomarker panel. To date, large scale (exome) sequencing studies in bladder cancer have focused on late stage muscle invasive disease. As part of our efforts to increase the comprehensive nature of our panel across clinical subtypes we include TERT promoter, FGFR3 and STAG2 mutations, all of which are significantly more prevalent in low grade disease. Previous exome sequencing studies do not capture TERT promoter mutations, a highly prevalent biomarker present in 70-80% of bladder cancer patients ((C. D. Hurst, F. M. Platt, and M. A. Knowles, "Comprehensive Mutation Analysis of the TERT Promoter in Bladder Cancer and Detection of Mutations in Voided Urine," Eur. Urol); (P. J. Killela, Z. J. Reitman, Y. Jiao, C. Bettegowda, N. Agrawal, L. A. Diaz, A. H. Friedman, H. Friedman, G. L. Gallia, B. C. Giovanella, A. P. Grollman, T.-C. He, Y. He, R. H. Hruban, G. I. Jallo, N. Mandahl, A. K. Meeker, F. Mertens, G. J. Netto, B. A. Rasheed, G. J. Riggins, T. A. Rosenquist, M. Schiffman, I.-M. Shih, D. Theodorescu, M. S. Torbenson, V. E. Velculescu, T.-L. Wang, N. Wentzensen, L. D. Wood, M. Zhang, R. E. McLendon, D. D. Bigner, K. W. Kinzler, B. Vogelstein, N. Papadopoulos, and H. Yan, "TERT promoter mutations occur frequently in gliomas and a subset of tumors derived from cells with low rates of self-renewal," Proc. Natl. Acad. Sci., vol. 110, no. 15, pp. 6021-6026, Apr. 2013); (X. Liu, G. Wu, Y. Shan, C. Hartmann, A. von Deimling, and M. Xing, "Highly prevalent TERT promoter mutations in bladder cancer and glioblastoma," Cell Cycle, vol. 12, no. 10, pp. 1637-1638, May 2013); and (I. Kinde, E. Munari, S. F. Faraj, R. H. Hruban, M. Schoenberg, T. Bivalacqua, M. Allaf, S. Springer, Y. Wang, L. A. Diaz, K. W. Kinzler, B. Vogelstein, N. Papadopoulos, and G. J. Netto, "TERT promoter mutations occur early in urothelial neoplasia and are biomarkers of early disease and disease recurrence in urine," Cancer Res., vol. 73, no. 24, pp. 7162-7167, Dec. 2013)). In addition to an expansion and optimization of panel design, in certain embodiments we transition from amplicon sequencing to a hybrid capture library preparation approach. Hybrid capture reagents provide more uniform coverage across our targets, enhanced genomic complexity in our library, greater ability to computationally mark duplicates, fewer PCR cycles and reduced polymerase introduced error, and reduced library preparation costs allowing affordable deeper sequencing, with any one or more of these advantages contributing to enhanced assay sensitivity.

### Example 10

### (Development of error suppression methodologies to permit sensitive and specific urine based genome monitoring)

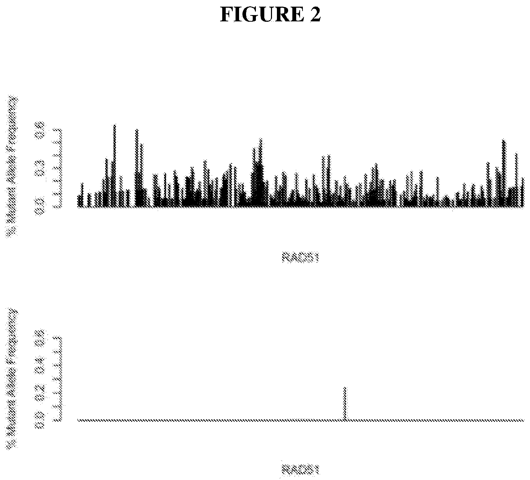

Traditional NGS methods produce substantial noise which limits detection of allele variants below 1-5%. In Fig. 2, we demonstrate a standard level of noise across nucleotides within the cancer gene Rad51. Even with PCR free methods, at a level below 0.6% mutant allele frequency almost all nucleotides demonstrate a level of non-reference reads when sequenced at 5,000x depth. Using our error suppression methods and high efficiency library conversion, we are able to detect true positive events from spike in studies without detection of standard noise (right, bottom). These analytical and library preparation enhancements lend themselves to development of diagnostic methods which track low frequency disease causing genomic abnormalities over time.

### Certain Computer Processor Based Embodiments

In certain embodiments, the steps described and/or performed hereinabove can be implemented by and in numerous ways, including without limitation, as one or more systems or apparatuses; one or a plurality of processes; a composition of matter; a series of instructions resident or non-resident to one, or a plurality of hardware devices coupled and/or in communications together; one or a plurality of computer program products being tangibly embodied on a computer readable storage medium and operable upon on one or more processors; any one or more processor configured to execute instructions provided by a memory coupled to the processor; and any technologies known to skilled persons involving the reading and/or execution of instructions by machines. Unless stated otherwise, a component such as a processor or a memory described as being configured to perform a task may be implemented as a general component that is temporarily configured to perform the task at a given time or a specific component that is manufactured to perform the task. As used herein, the term "processor" refers without limitation to one or more devices, circuits, processing cores or other instructions being executed by one, or a plurality of machines communicatively coupled together, and may be configured to process resident or non-resident data in any form.

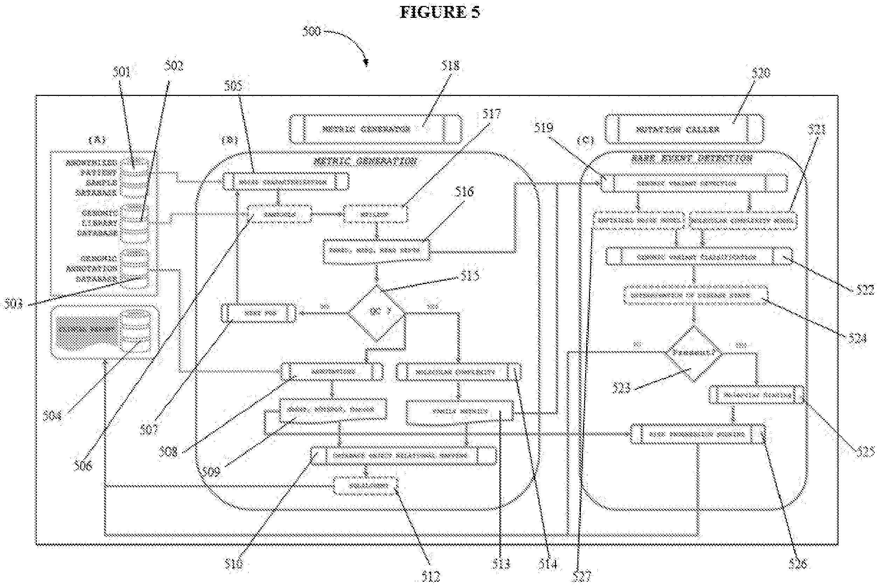

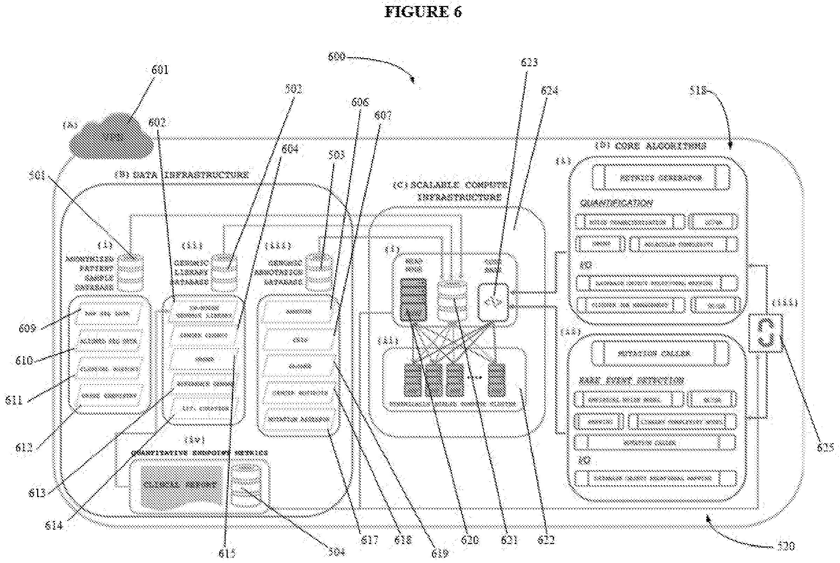

Now referring to Figs. 5 and 6, an illustration of a Flow Algorithm comprising (A) Genomic libraries 502 and 503 and raw patient sequencing data 501 serve as inputs to the algorithm is disclosed. In some embodiments, the genomic libraries are composed of proprietary in-house genomic libraries 602, and data collected from open sources including the Sanger Cosmic Database 604 (http://cancer.sanger.ac.uk/cosmic), dbSNP (www.ncbi.nlm.nih.gov/projects/SNP/), reference genome information 613, e.g. (http://hgdownload.cse.ucsc.edu/goldenPath/hg19/chromosomes/), and a curation of available scientific literature 614. In some embodiments, the genomic libraries composed of annotations 503. These are open-source libraries curated from scientific literature. These include Annovar (PMC2938201) 606, the CBIO portal (www.cbioportal.org/) 607, OncoKB (www.oncokb.org) 619, the Cancer Hotspots project (http://cancerhotspots.org/) 618, and Mutation Assessor (http://mutationassessor.org/r3/) 617. In some embodiments, the raw patient sequencing data input comprises an anonymized patient sample database 503 containing raw (unprocessed) sequencing data 609, aligned sequencing data 610, clinical history of the patient 611, and a urine chemistry profile of the urine 612. (B) The metrics generator 518 extracts 50 metrics. These metrics are generated by filtering read information on quality measures Phred, MAPQ, and Read Depth 516 computed using SAMTOOLS 506 (www.htslib.org). In some embodiments, the metrics generator comprises a Noise characterization program 505. Moreover, in order to determine the quality of genomic measurements in patient sample data SAMTOOLS in conjunction with the multi-way pileup command 517 is used to quantify the phred score (PHRED - a statistic defining sequencer base call confidence), mapping quality (MAPQ - a statistic defining a genomic alligner's confidence in read mapping), and read depth (READ DEPTH - metric defining the number of times a location was measured or counted) at each loci within the patient genes of interest. Then, these data are passed to our quality control filter 515. The filter performs a series of logic arguments to insure metrics are defined within expected ranges. If poorly measured or poorly mapped genomic information is encountered this information is discarded and the algorithm moves to a new position to determine noise characteristics. If QC criteria are met the next step in the algorithm begins. If reads possess quality information the molecular complexity 514 of the reads is determined (termed Family Metrics 513) and combined with annotations 508 from open source data tools including dbSNP, Cancer Hotspots, and OncoDB 509. These data are then persisted to our database for reporting. Quantifying molecular complexity provides a measure of successful library preparation and a sequencing methods ability to adequately sample a library of nucleic acids. Molecular complexity 514 is quantified by the number of distinct measurements among sequencing reads. During library preparation individual molecules are copied producing families of duplicate molecules - termed Families. In turn, each family corresponds to a unique molecule used to identify the base at the current location in the genome. The number of families provides a basis for understanding the number of unique molecules used to verify the presence of a given base and/or mutational event. Family metrics 513 are further used to suppress sequencing and PCR amplification induced errors. Annotations are comprised of databases of previously characterized genomic events describing normal human variation, catalogued cancer variation, or algorithims that predict variant function based on mutation type, location within a gene, resultant change to protein structure, and other criteria to estimate the mutational events relevance to basic cellular processes or pathological cellular processes in relation to cancer or risk of developing cancer. The number and type of annotations at a given base position provide in part a means to define the oncogenic nature of a given mutation. This information is used to inform molecular grading and clinical reporting. (C) The 50 metrics plus annotations determined by Metrics Generator serve as the input to the Mutation Caller 520 which filters these data to classify genomic variants. Genomic variant detection 519 is the process of quantifying the degree to which measured patient DNA is discordant with the healthy human genome and using metrics to help distinguish noise (false positive variants) from true positive genomic variants. This discordance is quantified by placing statistical thresholds on the amount of empirical noise - defined as the degree to which a base is difficult or easy to measure, the amount of high quality data present - and this discordance can be further supported or refuted by the amount of unique molecules that comprise a population of measurements at a given base location. Empirical noise is modeled specifically for nucleic acids extracted and sequenced from urine. Empirical noise models 527 may also integrate various patient clinical features and clinical chemical measurements within the urine. Error profiles are created to encompass the physiologic and pathologic diversity of urine samples, generated from clinically annotated urine genomic samples. Molecular complexity models 521 are generated through combinations of the molecular complexity metrics into an algorithm. These variants, if present, are further classified according to their molecular grading 525 and compared against both clinical data for the patient and previous characterization of the genomic event among Cancer Hotspots, OncoDB, TCGA. Genomic variant classification 522 comprises a method by which an algorithm is applied using the values generated in the Metric Generator process where these values comprise both a combination of empirical noise modeling 527 and molecular complexity modeling 521 (See Algorithm Flow Diagram Fig 5. Where the combination and thresholds for these metrics are determined through use of urine specific reference samples and serial dilutions and/or matched tumor-urine correlation studies to optimize technical sensitivity, specificity, true positive, false positive, true negative 523 and false negative rates and for the determination of a disease state 524. And where these combinations and thresholds are further refined through use of iterative testing and machine learning algorithms such as random forest. Molecular grading 525 is a process where variant annotation, reference to prior curated literature, genomic variation databases (in house and public), as well as algorithms based on unique combinations of various genomic variants within a patient and their correlation to clinical features and/or traditional pathologic grade. Molecular grade may be classified and reported in similar fashion to traditional pathologic grading, such as high or low grade. Molecular grade may also provide prognostic information related to risk of progression, recurrence, or risk of future tumor development in a symptomatic or unsympotomatic individual who is normal or at elevated risk for development of cancer. Risk progression scoring 526 may be provided if an individual is being monitored for cancer recurrence a risk progression score may be assigned based on the combination of molecular and clinical features. Together, these metrics form the basis of clinical reporting. The Flow Algorithm comprises a program for saving data to a database, whereby a database object relational mapping 510 provides a means to store program variable to a table structure or file structure in a database. In some embodiments, the means to store program variable to a table structure or file structure in a database is provided by an open source tool that provides this functionality including but not limited to SQLAlchemy 512. The outputs from the algorithms described are combined with clinical data into a clinical report 504. These are persisted to a sharable data repository for use by company researchers and partner physicians.

In certain embodiments, the methods of any one or combination multiple embodiments herein, are instructed by a computer-readable medium having stored thereon computer-readable instructions for carrying out such methods.

Turning back to the drawings, Fig 6 illustrates (A) a computational platform which is cloud-based computing infrastructure contained in the virtual private network (VPN) 601. The computational platform is supported by a data infrastructure (B) comprised of both proprietary (B i, ii, iv) 501,502 and 504 and open source (B iii) 503 genomic libraries. These libraries in conjunction with raw patient sequencing data (B i) 501 serve as the inputs to the computational algorithms (D I, ,ii) 518 and 520 that extract a pluarlity of different metrics for the characterization of genetic mutations. The data processing is carried out in parallel on a dynamically scaled computer cluster (C i, ii) 624 that outputs data to a central data repository 621. The compute cluster may comprise a network of computers 622 that receives compute jobs from the central computer as well as input data and the code base 623 to carry out computation. Each computer in the cluster performs computation and returns data to the shared storage of the central computer 620. This data repository is used to both disseminate findings (B iv) 504, catalog mutations for further refinements of mutation detection (B ii, D iii) 502 and 503. The code base (D) 623 is comprised of three fundamental components: metric extraction from patient sequencing data and genomic libraries (D i) 518, genetic mutation quantification (D ii) 520 and the refinement of these approaches in the presence of calculated mutation signatures (D iii) 625.

The above described execution of instructions in any and all of the foregoing manners of execution, are employed in reference to: analysis algorithms being implemented in the improved assay that may allow, for example, longitudinal monitoring of urine DNA following initial assessment of a patient's primary tumor or following longitudinal analysis of multiple urine DNA nucleic acid samples; developing an enhanced targeted panel of biomarkers that, for example, are capable of encompassing the genomic and clinical diversity of a bladder cancer, and in certain embodiments hematuria; in certain embodiments providing high technical performance while simultaneously achieving clinically feasible assay costs and processing times; monitoring the urine of bladder patients in a manner that yield high sensitivity and specificity; detecting mutations in one or more genes associated with bladder cancer; isolating nucleic acid, DNA or RNA, from a urine sample from a subject, and analyzing the nucleic acid to obtain nucleic acid sequence data suitable to detect presence or absence of one or more mutations in one or more of genes associated with bladder cancer; isolating nucleic acid being cell-free nucleic acid and/or being nucleic acid isolated from cells in a urine sample; and/or performing one or more of the methods cited herein in relation to an individual or group of individuals for detection, prognosis, diagnosis and treatment of bladder cancer in accordance with the embodiments including without limitation via use of genetic biomarkers and methodologies in gene sequencing.

In exemplary embodiments, a sequence or other data, is input to a processor or other computer hardware component. Here, the processor is coupled or otherwise in communication with a sequencing device that reads and/or analyzes sequences of nucleic acids from samples. The sequences are provided from processing tools or from sequence storage sources. One or more memory devices buffers or stores the sequences. The memory can also store reads, tags, fragments, phase information and islands, etc., for various chromosomes or genomes, and can store instructions for analyzing and presenting the sequence or aligned data.

In certain embodiments, the methods also include collecting data regarding a plurality of nucleotide sequences. Examples include reads, tags and/or reference chromosome sequences. The data can be sent to a processing device, hardware system or other computational system. In an exemplary embodiment, processor is connected to laboratory equipment. Such equipment can include a nucleotide amplification means, a sample collection means, nucleotide sequencing means and/or a hybridization means.

The processor can then collect applicable data having been gathered by the laboratory device. In exemplary embodiments, not to be taken as an exhaustive list, the data is stored in resident or non-resident storage means of a machine or other processing apparatus; the data is collected in real time, prior, during or in conjunction with the transmission of the data; the data is stored on a computer-readable medium that is extractable from the processor; the data is transmitted to a remote location via any means of coupling or communications, including without limitation, via a computer bus, via a local area network, via a wide area network, over an Intranet or the Internet, via wireline, wireless or satellite signals, and over any known form or media of transmission; the data is processed and operated upon at the remote location.

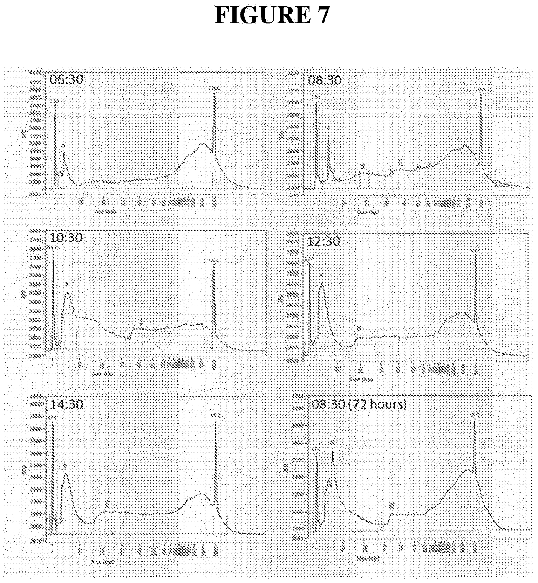

Now referring to Fig. 7, a capillary electrophoresis profiles of urine nucleic acid collected from the same individual through the course of the day is shown. The relative size of the nucleic acid is defined by lower and upper marker reference peaks (LM, UM) where LM represents 0bp and UM represents 5,000bp (horizontal axis, size in base pairs). Vertical Axis reflects the fluorescence intensity value of a nucleic acid binding dye which permits quantification of the molarity of nucleic acid molecules at a particular size.

As reflected by Fig. 7 early morning voids are most often characterized by a large amount of high molecular weight nucleic acid and a low abundance of small molecules. In this state these nucleic acid populations are separated and urine has typically spent the greatest period of time in contact with the urologic tract and bladder, enriching the abundance of urologic tract biomarkers in urine. As the day progresses increased intermediate sized molecules appear due to increased degratory conditions within urine or altered kidney physiology modifying the size distribution of the trans-renal DNA fraction. Degradation prone conditions are further characterized by a downward shift (towards the lower marker) of the high molecular weight peak, and a broadening and increase in intensity of the smaller lower molecular weight peak. In cases where total DNA measurements are made, smaller (less than 80bp) molecules can dilute the signals from urologic tract nucleic acid and compromise sequencing library preparation efficiency if not normalized for accordingly.

According to an embodiment of a method of the invention, urine is collected from morning void urine samples to aid enrichment of urologic tract signals and the urine is processed according to one or more techniques disclosed herein. Next, the nucleic acid markers and or other markers are analyzed. Data regarding the time of the collection is collected, as well as other patient data including identity, age, weight, gender, medications, diseases, clinical and other personal data, and such is tracked with the sample and entered into a database. In embodiments the resulting data is compared with same patient data at the same collection time and at different collection times. In other embodiments, the data is compared with other patient data. Depending on capillary electrophoresis and/or real time PCR quality control the quantification and normalization of the data is performed and a DNA library is constructed using size profile information.

The variability in DNA fragmentation profiles may be caused by diverse physiology and storage conditions. In addition to the time of day collected, certain individuals appear to have natural biases to one urine profile type over another. In individuals with a predominantly small/trans-renal profile it is of further importance to collect samples when urine incubation with the bladder has been maximized (early morning) and in other cases immediate voiding of urine into a preservation buffer which inhibits nuclease activity to prevent degradation of nucleic acid.

The heterogeneity of nucleic acid size in urine is described across a representative sampling of people of various age, gender, and disease or wellness states by using capillary electrophoresis and/or analysis of sequencing read start and stop site analysis. Using this data, it is possible to assess if and how urine nucleic acid size and fragmentation profiles change within an individual over the course of time (hours, days, weeks, or months) and in response to physiologic perturbations such as disease, circadian rhythm, diet, and hydration. In a version of this embodiment, nucleic acid size and fragmentation patterns within urine are used as one component of a disease classifying algorithm.

Additionally, it has been determined that sample handling, preservation and storage conditions will influence molecules of various size or the heterogeneity observed within a particular urine sample. In addition, nucleic acid extraction methods also influence size variability in samples had not been previously characterized in connection with such urine analysis. As discussed below particular patient's sampled also manifest different nucleic acid size profiles. While the ultimate impact of various size profiles on sequencing library preparation efficiency and sequencing performance has not been completely characterized, embodiments of the present invention involve the characterization of each of these variables and then data collected is used to create a database of patient profiles and ultimately improve both the diagnoses and prognosis of bladder cancer. In embodiments, data collected relating to the nucleic acid size is associated with one or more of the various correlating factors discussed above. In an embodiment, samples from patients with predetermined profiles are normalized according to their profile and assessed by sequencing to measure unique fragmentation (sequencing start/stop) sites and where this sequence context fragmentation pattern is integrated as one aspect into a disease diagnosis algorithm.

It has been determined that urine nucleic acid has substantial heterogeneity in its size distribution across individuals. Further, the heterogeneity of the nucleic acids in the sample size is not uniform within individuals throughout the day. In addition, nucleic acid degradation in samples can substantially reduce the size of nucleic acid molecules when urine is left at room temperature for minutes to hours to days. Degradation may occur in various ways, in one example higher molecular weight DNA degrades, becoming smaller in size and increasing the abundance of small molecular weight DNA within the urine, referred to as low molecular weight pooling. In another example, high molecular weight DNA completely degrades beyond detection and does not accumulate within a low molecular weight pool. Additionally, the process of freezing and defrosting urine has substantial impact on nucleic acid size and damage. In one embodiment, degradation of DNA due to handling damage can be distinguished from DNA fragmented due to biologic processes such as apoptosis and necrosis through analysis of sequence context around read start and stop bases. And where this information is used to create a sample quality ratio to normalize sequencing data.

According to an aspect of the invention, a database is developed that includes various nucleic acid size profiles and fragmentation sequence context analysis across thousands of unique urine samples and across hundreds of unique physiologies, pathologies, and treatment conditions. This data is then correlated with the sampling data and the records are compared to provide outputs that relate to the underlying causes for variable urine nucleic acid size.

Embodiments of the present invention involve the steps of (1) iterative optimization of sequencing methodologies to various size profiles, (2) optimizing sample collection and storage techniques to maintain integrity of nucleic acid size, (3) the implementation of quality controls to filter out samples of poor quality, and (4) the normalization of final sequencing data back to unique features of nucleic acid size profiles. Taken together, these steps and multiple iterations on sequencing methods have led to a high quality urine based genomics analysis.

The diagnostic sensitivity for detection of diseases within the urologic tract is influenced by the size distribution of nucleic acids in the sample. Based on this understanding, we have defined the following parameters/combinations to enhance assay performance.

In an embodiment, an analysis includes the targeting and enrichment of nucleic acids in the 120-5000bp range and/or in the 5,000-10,000bp range depending on sample profile and wherein these size ranges may be fragmented to population of molecules that are 500-600bp in size through (1) mechanical fragmentation techniques such as ultasonication disclosed by Covaris, (2) enzyme based fragmentation, such as that performed by Kapa Hyper-plus, (3) restriction enzyme or (4) a cocktail of various restriction enzymes and wherein fragmented molecules are then placed into a library preparation reaction.

In an embodiment, an analysis of urine samples that are collected from an individual during the first or second morning void, thereby maximizing the time the urine has spent in contact with bladder epithelium is conducted. After sample processing using fragmentation techniques, the analysis of the genome is performed to determine if a plurality of marker DNA or RNA marker segments are present in the sample.

In an embodiment, an analysis of urine samples collected prior to consuming a meal or drinking fluids, minimizing physiologic activity of the kidney, wherein said analysis comprises processing to determine if a plurality of marker DNA or RNA segments are in the sample.

In an embodiment, the normalization of the sample, to size of nucleic acids is performed (1) to develop a urine sequencing diagnostic that analyzes signals in urologic tract it is favorable to enrich for and analyze nucleic acid that is greater than 100-150bp in size and (2) to develop a urine sequencing diagnostic that analyzes nucleic acid signals from systemic circulation it is favorable to enrich nucleic acid that is smaller than 100 base pairs in size and specifically may range from 20-100 base pairs depending on kidney function/health. Common DNA measurements such as UV absorption and fluorimetry do not provide size information and may cause over or under loading of DNA into a library preparation reaction if used in isolation (see Figure 3). For this reason, methods of the invention use in combination capillary electrophoresis and/or a real time PCR reaction where the primers of the reaction are designed to be specific nucleotide distances apart to measure the abundance of one size relative to another. There may be multiple (1, 2, 3) sets of primers/amplicons designed for different sizes (for example Kapa human DNA quantification kit). These primers may be designed for amplicons 30-70 base pairs in size, 70-150 base pairs in size, 150-500 base pairs in size or greater than 1,000 base pairs in size. Upon determining the size profile of a nucleic acid sample from urine, the sample may be normalized to insure sufficient quantiles of a specific size range are placed into a library preparation reaction. In one embodiment, this would be molecules greater than 80bp in size (See Figure 4). Based on total nucleic acid loading, subsequent library preparation steps may be modified such as the volume of carboxylated para-magnetic bases used in clean up after ligation. Alternatively, prior to library preparation, nucleic acid may be differentially separated based on fragment size by passing over a size selection column (such as Pall Nanosep^{®} device), treatment with carboxylated para-magnetic beads (such as AmpPureXP Beads), capillary gel electrophoresis, gel electrophoresis or anion exchange (Such as Sage Sciences Pippen Prep or Pall Mustang Membrane).

Fig. 9 depicts dot plot graphs that represent the relationship between allele frequency in tumor and urine nucleic acid in patient matched samples where urine was collected while tumor was present in the bladder. The vertical axis is non-reference allele frequency, horizontal axis is genomic position within targeted genomic region, the dots denote sample type and are described in figure key. Patient A shows that some patients have high allele frequency concordance between tumor (range 42-71%) and urine (38-60%), where the majority of nucleic acid in urine is of tumor origin. Conversely, patient B shows another scenario where the abundance of tumor derived nucleic acid in urine is much lower and tumor (26-51%) and urine (0.3-2.2%) mutation abundance is discordant while urine mutations still maintain abundance above reference database ranges for those positions (grey X). Both Patient A and B demonstrate an additional characteristic of distinct allele frequency clusters within both tumor and urine samples. In one embodiment the extent or type of allele frequency clustering may be used as part of a diagnostic or prognostic disease algorithm.

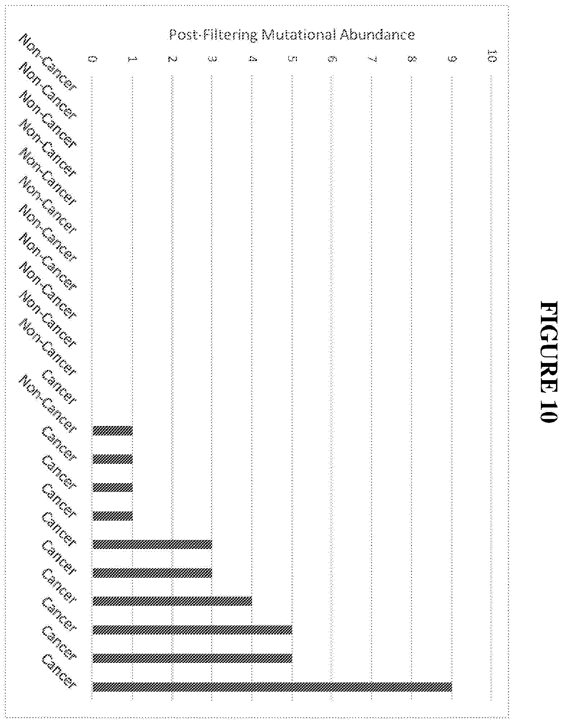

Fig. 10 depicts a bar graph of filtered mutational abundance in non-cancer and cancer patients. Urine was collected from patients where cancer or no cancer was known to exist within their urologic tract. The horizontal axis represents unique patient urine samples, while the vertical axis represents the number of mutations identified in a sample following algorithmic filtering (described in figure 5 & 6) when this analysis is performed on 40 preselected genes (taken from table 1). Upon implementation of the quality controls and data analysis algorithms described herein, the number of putative mutations within these samples is adjusted from between 133-1,984 events, to that shown here, 0-9 events per sample. Post filtering, the abundance of urine associated mutations is able to separate diseased from a non-disease state and has utility for diagnosis, detection of recurrent disease, and disease characterization.

### Conclusion

It should be noted that the depicted order and labeled operations herein are indicative of one or more exemplary embodiments of certain presented methods. Other operations and methods can be conceived by skilled persons that are equivalent in function, logic, or effect to one or more operations, or portions thereof, of the illustrated methods. Although the operations of the methods herein are shown and described in a particular order, the order of the operations of each method may be altered so that certain operations may be performed in an inverse order or so that certain operations may be performed, at least in part, concurrently with other operations. In other embodiments, instructions or sub-operations of distinct operations may be implemented in an intermittent and/or alternating manner.

Lastly, while various embodiments of the present invention have been described above, it should be understood that they have been presented by way of example only, and not limitation. Thus, the breadth and scope of the present embodiments should not be limited by any of the above-described description.

### Literature Cited

[17] A. M. Newman, S. V. Bratman, J. To, J. F. Wynne, N. C. W. Eclov, L. A. Modlin, C. L. Liu, J. W. Neal, H. A. Wakelee, R. E. Merritt, J. B. Shrager, B. W. Loo Jr, A. A. Alizadeh, and M. Diehn, "An ultrasensitive method for quantitating circulating tumor DNA with broad patient coverage," Nat. Med., vol. advance online publication, Apr. 2014.
[18] S. R. Kennedy, M. W. Schmitt, E. J. Fox, B. F. Kohrn, J. J. Salk, E. H. Ahn, M. J. Prindle, K. J. Kuong, J.-C. Shen, R.-A. Risques, and L. A. Loeb, "Detecting ultralow-frequency mutations by Duplex Sequencing," Nat. Protoc., vol. 9, no. 11, pp. 2586-2606, Nov. 2014.
[19] M. W. Schmitt, S. R. Kennedy, J. J. Salk, E. J. Fox, J. B. Hiatt, and L. A. Loeb, "Detection of ultra-rare mutations by next-generation sequencing," Proc. Natl. Acad. Sci. U. S. A., vol. 109, no. 36, pp. 14508-14513, Sep. 2012.
[20] E. Crowley, F. Di Nicolantonio, F. Loupakis, and A. Bardelli, "Liquid biopsy: monitoring cancer-genetics in the blood," Nat. Rev. Clin. Oncol., vol. 10, no. 8, pp. 472-484, Aug. 2013.
[21] M. Murtaza, S.-J. Dawson, D. W. Y. Tsui, D. Gale, T. Forshew, A. M. Piskorz, C. Parkinson, S.-F. Chin, Z. Kingsbury, A. S. C. Wong, F. Marass, S. Humphray, J. Hadfield, D. Bentley, T. M. Chin, J. D. Brenton, C. Caldas, and N. Rosenfeld, "Non-invasive analysis of acquired resistance to cancer therapy by sequencing of plasma DNA," Nature, vol. 497, no. 7447, pp. 108-112, May 2013.
[22] T. Forshew, M. Murtaza, C. Parkinson, D. Gale, D. W. Y. Tsui, F. Kaper, S.-J. Dawson, A. M. Piskorz, M. Jimenez-Linan, D. Bentley, J. Hadfield, A. P. May, C. Caldas, J. D. Brenton, and N. Rosenfeld, "Noninvasive Identification and Monitoring of Cancer Mutations by Targeted Deep Sequencing of Plasma DNA," Sci. Transl. Med., vol. 4, no. 136, pp. 136ra68-136ra68, May 2012.
[23] G. Sozzi, D. Conte, M. Leon, R. Ciricione, L. Roz, C. Ratcliffe, E. Roz, N. Cirenei, M. Bellomi, G. Pelosi, M. A. Pierotti, and U. Pastorino, "Quantification of free circulating DNA as a diagnostic marker in lung cancer," J. Clin. Oncol. Off. J. Am. Soc. Clin. Oncol., vol. 21, no. 21, pp. 3902-3908, Nov. 2003.
[24] C. Fernandez, Shore, and A. Shuber, "Noninvasive multianalyte diagnostic assay for monitoring bladder cancer recurrence," Res. Rep. Urol., p. 49, Oct. 2012.
[25] C. Fernandez, Millholland, Li, and A. Shuber, "Detection of low frequency FGFR3 mutations in the urine of bladder cancer patients using next-generation deep sequencing," Res. Rep. Urol., p. 33, Jun. 2012.
[29] W. Ranasinghe and R. Pers, "The Changing Incidence of Carcinoma In-Situ of the Bladder Worldwide," in Advances in the Scientific Evaluation of Bladder Cancer and Molecular Basis for Diagnosis and Treatment, R. Persad, Ed. InTech, 2013.
[31] S. Myllykangas, J. D. Buenrostro, G. Natsoulis, J. M. Bell, and H. P. Ji, "Efficient targeted resequencing of human germline and cancer genomes by oligonucleotide-selective sequencing," Nat. Biotechnol., vol. 29, no. 11, pp. 1024-1027, Nov. 2011.
[32] H. Lee, B. T. Lau, and H. P. Ji, "Targeted Sequencing Strategies in Cancer Research," in Next Generation Sequencing in Cancer Research, W. Wu and H. Choudhry, Eds. Springer New York, 2013, pp. 137-163.
[33] "Press Announcements - FDA allows marketing of four 'next generation' gene sequencing devices." [Online]. Available: www.fda.gov/NewsEvents/Newsroom/PressAnnouncements/ucm375742.htm. [Accessed: 02-Dec-2014].
[34] K. Bijwaard, J. S. Dickey, K. Kelm, and Z. Težak, "The first FDA marketing authorizations of next-generation sequencing technology and tests: challenges, solutions and impact for future assays," Expert Rev. Mol. Diagn., pp. 1-8, Nov. 2014.
[35] F. S. Collins and M. A. Hamburg, "First FDA Authorization for Next-Generation Sequencer," N. Engl. J. Med., vol. 369, no. 25, pp. 2369-2371, Nov. 2013.
[36] D. C. Koboldt, Q. Zhang, D. E. Larson, D. Shen, M. D. McLellan, L. Lin, C. A. Miller, E. R. Mardis, L. Ding, and R. K. Wilson, "VarScan 2: somatic mutation and copy number alteration discovery in cancer by exome sequencing," Genome Res., vol. 22, no. 3, pp. 568-576, Mar. 2012.
[37] A. Wilm, P. P. K. Aw, D. Bertrand, G. H. T. Yeo, S. H. Ong, C. H. Wong, C. C. Khor, R. Petric, M. L. Hibberd, and N. Nagarajan, "LoFreq: a sequence-quality aware, ultra-sensitive variant caller for uncovering cell-population heterogeneity from high-throughput sequencing datasets," Nucleic Acids Res., vol. 40, no. 22, pp. 11189-11201, Dec. 2012.
[38] Z. Wei, W. Wang, P. Hu, G. J. Lyon, and H. Hakonarson, "SNVer: a statistical tool for variant calling in analysis of pooled or individual next-generation sequencing data," Nucleic Acids Res., vol. 39, no. 19, p. e132, Oct. 2011.
[39] K. Cibulskis, M. S. Lawrence, S. L. Carter, A. Sivachenko, D. Jaffe, C. Sougnez, S. Gabriel, M. Meyerson, E. S. Lander, and G. Getz, "Sensitive detection of somatic point mutations in impure and heterogeneous cancer samples," Nat. Biotechnol., vol. 31, no. 3, pp. 213-219, Mar. 2013.
[41] J. Reading, R. R. Hall, and M. K. Parmar, "The application of a prognostic factor analysis for Ta.T1 bladder cancer in routine urological practice," Br. J. Urol., vol. 75, no. 5, pp. 604-607, May 1995.

**TABLE 1**

| Gene Symbol | Synonym Symbol | Full Gene Name | Chromosome | HG19 Basepair Start Site | HG19 Basepair Stop Site |
|---|---|---|---|---|---|
| KDM6A | | lysine (K)-specific demethylase 6A | X | 44732423 | 44971845 |
| MLL2 | KMT2B | lysine (K)-specific methyltransferase 2D | 12 | 49412758 | 49449107 |
| TSC1 | | tuberous sclerosis 1 | 9 | 135766735 | 135820020 |
| NOTCH2 | | notch 2 | 1 | 120454176 | 120612317 |
| PTEN | | phosphatase and tensin homolog | 10 | 89623195 | 89728532 |
| TP53 | | tumor protein p53 | 17 | 7571720 | 7590868 |
| NOTCH1 | | notch 1 | 9 | 139388896 | 139440238 |
| CDKN2A | | cyclin-dependent kinase inhibitor 2A | 9 | 21967751 | 21994490 |
| RB1 | | retinoblastoma 1 | 13 | 48877883 | 49056026 |
| ATM | | ATM serine/threonine kinase | 11 | 108093559 | 108239826 |
| ERBB2 | | erb-b2 receptor tyrosine kinase 2 | 17 | 37844393 | 37884915 |
| PIK3CA | | phosphatidylinositol-4,5-bisphosphate 3-kinase, catalytic subunit alpha | 3 | 178866311 | 178952497 |
| FGFR3 | | fibroblast growth factor receptor 3 | 4 | 1795039 | 1810599 |
| EGFR | | epidermal growth factor receptor | 7 | 55086725 | 55275031 |
| FGFR1 | | fibroblast growth factor receptor 1 | 8 | 38268656 | 38326352 |
| CREBBP | | CREB binding protein | 16 | 3775056 | 3930121 |
| LRP1B | | low density lipoprotein receptor-related protein 1B | 2 | 140988996 | 142889270 |
| MYC | | v-myc avian myelocytomatosis viral oncogene homolog | 8 | 128748315 | 128753680 |
| ARID1A | | AT rich interactive domain 1A (SWI-like) | 1 | 27022522 | 27108601 |
| MLL3 | KMT2C | lysine (K)-specific methyltransferase 2C | 7 | 151832010 | 152133090 |
| BIRC3 | | baculoviral IAP repeat containing 3 | 11 | 102188181 | 102210135 |
| WWOX | | WW domain containing oxidoreductase | 16 | 78133327 | 79246564 |
| PALB2 | | partner and localizer of BRCA2 | 16 | 23614483 | 23652678 |
| SOX4 | | SRY (sex determining region Y)-box 4 | 6 | 21593972 | 21598849 |
| YAP1 | | Yes-associated protein 1 | 11 | 101981192 | 102104154 |
| CCND1 | | cyclin D1 | 11 | 69455873 | 69469242 |
| BCL2L1 | | BCL2-like 1 | 20 | 30252261 | 30310656 |
| MYCL1 | | v-myc avian myelocytomatosis viral oncogene lung carcinoma derived homolog | 1 | 40361096 | 40367687 |
| MDM4 | | MDM4, p53 regulator | 1 | 204485507 | 204527248 |
| FGF3 | | fibroblast growth factor 3 | 11 | 69624736 | 69634192 |
| MDM2 | | MDM2 proto-oncogene, E3 ubiquitin protein ligase | 12 | 69201971 | 69239320 |
| CCNE1 | | cyclin E1 | 19 | 30302901 | 30315215 |
| ZNF703 | | zinc finger protein 703 | 8 | 37553301 | 37556396 |
| PRKCI | | protein kinase C, iota | 3 | 169940220 | 170023770 |
| NCOR1 | | nuclear receptor corepressor 1 | 17 | 15933408 | 16118874 |
| YWHAZ | | tyrosine 3-monooxygenase/tryptophan 5-monooxygenase activation protein, zeta | 8 | 101930804 | 101965623 |
| PPARG | | peroxisome proliferator-activated receptor gamma | 3 | 12329349 | 12475855 |
| TBL1XR1 | | transducin (beta)-like 1 X-linked receptor 1 | 3 | 176738542 | 176915048 |
| PDE4D | | phosphodiesterase 4D, cAMP-specific | 5 | 58264866 | 59783925 |
| IKZF2 | | IKAROS family zinc finger 2 (Helios) | 2 | 213864411 | 214016333 |
| SPAG1 | | sperm associated antigen 1 | 8 | 101170263 | 101254132 |
| E2F3 | | E2F transcription factor 3 | 6 | 20402137 | 20493945 |
| NIT1 | | nitrilase 1 | 1 | 161087862 | 161095235 |
| BEND3 | | BEN domain containing 3 | 6 | 107386385 | 107435636 |
| GDI2 | | GDP dissociation inhibitor 2 | 10 | 5807186 | 5855512 |
| PVRL4 | | poliovirus receptor-related 4 | 1 | 161040781 | 161059385 |
| CCSER1 | | coiled-coil serine-rich protein 1 | 4 | 91048684 | 92523370 |
| TERT Promoter | | telomerase reverse transcriptase promoter region | 5 | 1253287 | 1295162 |
| SPTAN1 | | spectrin, alpha, non-erythrocytic 1 | 9 | 131314837 | 131395944 |
| HRAS | | Harvey rat sarcoma viral oncogene homolog | 11 | 532242 | 535550 |
| CTNNB1 | | catenin (cadherin-associated protein), beta 1, 88kDa | 3 | 41240942 | 41281939 |
| FBXW7 | | F-box and WD repeat domain containing 7, E3 ubiquitin protein ligase | 4 | 153242410 | 153456393 |
| EP300 | | E1A binding protein p300 | 22 | 41488614 | 41576081 |
| RHOA | | ras homolog family member A | 3 | 49396579 | 49449526 |
| CCND3 | | ras homolog family member A | 6 | 41902671 | 42016610 |
| NOS1AP | | cyclin D3 | 1 | 162039581 | 162339813 |
| ELF3 | | nitric oxide synthase 1 (neuronal) adaptor protein | 1 | 201979690 | 201986315 |
| PTPRD | | E74-like factor 3 (ets domain transcription factor, epithelial-specific ) | 9 | 8314246 | 10612723 |
| STAG2 | | protein tyrosine phosphatase, receptor type, D | X | 123094475 | 123236505 |
| ERBB3 | | stromal antigen 2 | 12 | 56473809 | 56497291 |
| CDKN1A | | erb-b2 receptor tyrosine kinase 3 | 6 | 36644237 | 36655116 |
| NFE2L2 | | cyclin-dependent kinase inhibitor 1A (p21, Cip1) | 2 | 178095031 | 178129859 |
| AIRE | | nuclear factor, erythroid 2-like 2 | 21 | 45705721 | 45718102 |
| BTG2 | | autoimmune regulator | 1 | 203274664 | 203278729 |
| TTC28 | | BTG family, member 2 | 22 | 28374002 | 29075853 |
| IKZF3 | | tetratricopeptide repeat domain 28 | 17 | 37913968 | 38020441 |
| FHIT | | IKAROS family zinc finger 3 (Aiolos) | 3 | 59735036 | 61237133 |
| SHANK2 | | fragile histidine triad | 11 | 70313961 | 70935808 |
| ERCC2 | | SH3 and multiple ankyrin repeat domains 2 | 19 | 45854649 | 45873845 |
| TPTE | | excision repair cross-complementation group 2 | 21 | 10906743 | 10990920 |
| KLF5 | | transmembrane phosphatase with tensin homology | 13 | 73633142 | 73651676 |
| FOXA1 | | Kruppel-like factor 5 (intestinal) | 14 | 38058757 | 38064325 |
| PON3 | | forkhead box A1 | 7 | 94989184 | 95025687 |
| RXRA | | paraoxonase 3 | 9 | 137218316 | 137332431 |
| ZFP36L1 | | retinoid X receptor, alpha | 14 | 69254372 | 69262960 |
| GPC5 | | ZFP36 ring finger protein-like 1 | 13 | 92050935 | 93519487 |
| PCSK5 | | glypican 5 | 9 | 78505560 | 78977255 |
| CTIF | | proprotein convertase subtilisin/kexin type 5 | 18 | 46065427 | 46389586 |
| FOXQ1 | | CBP80/20-dependent translation initiation factor | 6 | 1312675 | 1314993 |
| TIMM9 | | forkhead box Q1 | 14 | 58875370 | 58894232 |
| CX3CL1 | | translocase of inner mitochondrial membrane 9 homolog (yeast) | 16 | 57406414 | 57418956 |
| TXNIP | | chemokine (C-X3-C motif) ligand 1 | 1 | 145438462 | 145442628 |
| RHOB | | thioredoxin interacting protein | 2 | 20646835 | 20649201 |
| PAIP1 | | ras homolog family member B | 5 | 43526370 | 43557521 |
| PHACTR1 | | ras homolog family member B | 6 | 12717037 | 13288075 |
| CDKAL1 | | poly(A) binding protein interacting protein 1 | 6 | 20534688 | 21232634 |
| TACC3 | | phosphatase and actin regulator 1 | 4 | 1723217 | 1746905 |
| ASXL2 | | CDK5 regulatory subunit associated protein 1-like 1 | 2 | 25962253 | 26101312 |
| HORMAD1 | | transforming, acidic coiled-coil containing protein 3 | 1 | 150670535 | 150693364 |
| PHLDA3 | | additional sex combs like transcriptional regulator 2 | 1 | 201434607 | 201438299 |
| MIPOL1 | | HORMA domain containing 1 | 14 | 37667118 | 38020464 |
| ZFR2 | | pleckstrin homology-like domain, family A, member 3 | 19 | 3804022 | 3869027 |
| PIGH | | mirror-image polydactyly 1 | 14 | 68056023 | 68067017 |
| WRB | | zinc finger RNA binding protein 2 | 21 | 40752213 | 40769815 |
| MRO | | phosphatidylinositol glycan anchor biosynthesis, class H | 18 | 48321490 | 48351754 |
| STYX | | tryptophan rich basic protein | 14 | 53196883 | 53241705 |
| MDFIC | | Maestro | 7 | 114562209 | 114659970 |
| ERMN | | serine/threonine/tyrosine interacting protein | 2 | 158175125 | 158184146 |
| RND3 | | MyoD family inhibitor domain containing ermin, ERM-like protein Rho family GTPase 3 | 2 | 151324707 | 151344209 |

The following numbered clauses contain further statements of various aspects of the present invention:-
1. A method for the diagnosis and/or monitoring the recurrence of bladder cancer in a subject, comprising:
   a) collecting a test sample selected from one or more urine samples and/or tumor samples containing a nucleic acid from a subject;
   b) isolating the nucleic acid collected from the one or more urine samples and/or tumor samples from the subject;
   c) analyzing the nucleic acid to obtain a nucleic acid sequence data; and
   d) determining a presence or an absence of at least one mutation and/or epigenetic alteration in at least one gene associated with bladder cancer in the nucleic acid, wherein the at least one mutation and/or epigenetic alteration in the at least one gene is/are selected from the mutations listed in Table 1.
2. The method of clause 1, wherein the the isolated nucleic acids are analyzed directly without an amplification step.
3. The method of clause 1, wherein the isolated nucleic acid is RNA, and wherein the RNAs are in certain embodiments reverse-transcribed into complementary DNAs, and wherein the reverse transcription may be performed alone or in combination with an amplification step.
4. The method of clause 1, wherein the isolated nucleic acids are analyzed directly without an amplification step.
5. The method of clause 1, wherein the nucleic acid is amplified for enrichment of known bladder cancer genes prior to the analyzing step c).
6. The method of clause 1, wherein the test sample comprises one or more urine samples.
7. The method of clause 1, wherein the test sample comprises one or more urine samples collected at different time points.
8. The method of clause 1, further comprises a computer-readable medium having computer executable instructions for determining a diagnosis and/or monitoring a recurrence of bladder cancer of a subject.
9. The method of clause 1, wherein the analysis step c) further comprises a computer-readable medium comprising one or more analysis algorithms.
10. The method of clause 1, further comprises determining the presence or an absence of one or more mutations in one or more of genes associated with bladder cancer from a genotype dataset derived from the subject.
11. The method of clause 1, wherein the nucleic acid in the or more samples is contacted with one or more reagents suitable for detecting the presence or absence of one or more mutations and/or epigenetic alterations in one or more genes associated with bladder cancer.
12. The method of clause 1, further comprises comparing the presence or absence of the one or more mutations and/or epigenetic alterations detected in a first urine sample nucleic acid to the presence or absence of the one or more mutations and/or epigenetic alterations detected in a second urine sample nucleic acid and monitoring the recurrence of bladder cancer in the subject based on the comparison.
13. The method of clause 1, wherein the at least one mutation in the at least one gene is detected using a hybridization assay, wherein the presence or absence of a gene mutation is determined based on the hybridization of one or more allele-specific oligonucleotide probes to one or more nucleic acid molecules in the DNA sample from the subject.
14. The method of clause 1, wherein the mutation in a gene is detected by sequencing technologies.
15. The method of clause 1, further comprises obtaining a first and a second urine samples containing the nucleic acid at different points in time from the subject.
16. A method for monitoring bladder cancer progression in a subject, comprising:
   a) collecting a test sample selected from one or more urine samples and/or tumor samples containing a nucleic acid from a subject;
   b) isolating the nucleic acid collected from the one or more urine samples and/or tumor samples from the subject;
   c) analyzing the nucleic acid to obtain a nucleic acid sequence data; and
   d) determining a presence or an absence of at least one mutation and/or epigenetic alteration in at least one gene associated with bladder cancer in the nucleic acid, wherein the at least one mutation and/or epigenetic alteration in the at least one gene is/are selected from the mutations listed in Table 1.
17. The method of clause 16, wherein the the isolated nucleic acids are analyzed directly without an amplification step.
18. The method of clause 16, wherein the isolated nucleic acid is RNA, wherein the RNAs are in certain embodiments reverse-transcribed into complementary DNAs, wherein the reverse transcription may be performed alone or in combination with an amplification step.
19. The method of clause 16, wherein the isolated nucleic acids are analyzed directly without an amplification step.
20. The method of clause 16, wherein the nucleic acid is amplified for enrichment of known bladder cancer genes prior to analyzing step c).
21. The method of clause 16, further comprises a computer-readable medium having computer executable instructions for determining cancer progression of a subject to bladder cancer.
22. The method of clause 16, wherein the analysis step c) further comprises a computer-readable medium comprising one or more analysis algorithms.
23. The method of clause 16, further comprises determining the presence or an absence of one or more mutations in one or more of genes associated with bladder cancer from a genotype dataset derived from the subject.
24. The method of clause 16, wherein the nucleic acid in the or more samples is contacted with one or more reagents suitable for detecting the presence or absence of one or more mutations and/or epigenetic alterations in one or more genes associated with bladder cancer.
25. The method of clause 16, further comprises comparing the presence or absence of the one or more mutations and/or epigenetic alterations detected in a first urine sample nucleic acid to the presence or absence of the one or more mutations and/or epigenetic alterations detected in a second urine sample nucleic acid and monitoring cancer progression in the subject based on the comparison.
26. The method of clause 16, wherein the at least one mutation in the at least one gene is detected using a hybridization assay, wherein the presence or absence of a gene mutation is determined based on the hybridization of one or more allele-specific oligonucleotide probes to one or more nucleic acid molecules in the DNA sample from the subject.
27. The method of clause 16, wherein the mutation in a gene is detected by sequencing technologies.
28. The method of clause 16, further comprises obtaining a first and a second urine samples containing the nucleic acid at different points in time from the subject.
29. A method for determining susceptibility of a subject to bladder cancer, comprising:
   a) collecting a test sample selected from one or more urine samples and/or tumor samples containing a nucleic acid from a subject;
   b) isolating the nucleic acid collected from the one or more urine samples and/or tumor samples from the subject;
   c) analyzing the nucleic acid to obtain a nucleic acid sequence data; and
   d) determining a presence or an absence of at least one mutation and/or epigenetic alteration in at least one gene associated with bladder cancer in the nucleic acid, wherein the at least one mutation and/or epigenetic alteration in the at least one gene is/are selected from the mutations listed in Table 1.
30. The method of clause 29, wherein the the isolated nucleic acids are analyzed directly without an amplification step.
31. The method of clause 29, wherein the isolated nucleic acid is RNA, wherein the RNAs are in certain embodiments reverse-transcribed into complementary DNAs, wherein the reverse transcription may be performed alone or in combination with an amplification step.
32. The method of clause 29, wherein the isolated nucleic acids, including DNA and/or RNA, are analyzed directly without an amplification step.
33. The method of clause 29, wherein the nucleic acid is amplified for enrichment of known bladder cancer genes prior to analyzing step c).
34. The method of clause 29, wherein the presence and/or a relative abundance of the at least one mutation is indicative of increased susceptibility to bladder cancer in the subject.
35. The method of clause 29, wherein the analysis step c) further comprises a computer-readable medium comprising one or more analysis algorithms.
36. The method of clause 29, further comprises a computer-readable medium having computer executable instructions for determining susceptibility of a subject to bladder cancer, wherein the computer-readable memory includes data indicative of the frequency of at least one mutation and/or epigenetic alteration of at least one gene in a plurality of individuals diagnosed with bladder cancer.
37. The method of clause 29, further comprises a computer-readable medium having computer executable instructions for determining susceptibility of a subject to bladder cancer, wherein the computer-readable memory includes data indicative of the frequency of at least one mutation and/or epigenetic alteration of at least one gene in a plurality of individuals diagnosed with bladder cancer, and wherein the risk measure for the subject can be based on a comparison of the genotype data set for the subject to the risk associated with the at least one mutation and/or epigenetic alteration of the at least one gene or the at least one genotype data set.
38. The method of clause 29, further comprises a computer-readable medium having computer executable instructions for determining susceptibility of a subject to bladder cancer, wherein the computer-readable memory includes data indicative of the frequency of at least one mutation and/or epigenetic alteration of at least one gene or at least one genotype data set in a plurality of individuals diagnosed with bladder cancer. The memory can also include data indicative of the frequency of at the least one mutation and/or epigenetic alteration of at least one gene or at least one genotype data set in a plurality of reference individuals.
39. The method of clause 29, further comprising determining the presence or absence of at least one or more mutations associated with bladder cancer in at least one or more genes in a genotype dataset derived from an individual or subject, wherein the determination of the presence and/or relative abundance of the at least one mutation is indicative of increased susceptibility to bladder cancer in the subject.
40. The method of clause 29, further comprises determining the presence or an absence of one or more mutations in one or more of genes associated with bladder cancer from a genotype dataset derived from the subject, wherein the genotype dataset may comprise allelic information about one or more mutations or epigenetic marker.
41. The method of clause 29, wherein the determination of the susceptibility comprises comparing the nucleic acid sequence data to a database containing correlation data between the at least one mutation and/or epigenetic marker and susceptibility to bladder cancer.
42. The method of clause 29, wherein the at least one mutation in the at least one gene is detected using a hybridization assay, wherein the presence or absence of a gene mutation is determined based on the hybridization of one or more allele-specific oligonucleotide probes to one or more nucleic acid molecules in the DNA sample from the subject.
43. The method of clause 29, wherein the mutation in a gene is detected by sequencing technologies.
44. A kit for assessing susceptibility to bladder cancer in a subject, comprising:
   a) one or more reagents necessary for selectively detecting at least one mutation and/or epigenetic alteration of at least one gene associated with bladder cancer in the genome of the subject, where the presence of the at least one mutation and/or epigenetic alteration is indicative of increased susceptibility to bladder cancer; and
   b) data including correlation data between the at least one mutation and/or epigenetic alteration and susceptibility to bladder cancer.
45. The kit of clause 44, wherein the one or more reagents include at least one contiguous oligonucleotide that hybridizes to a fragment of the genome of the individual including the at least one mutation.
46. The kit of clause 44, wherein the at least one pair of oligonucleotides that hybridize to opposite strands of a genomic segment obtained from the subject, where each oligonucleotide primer pair is designed to selectively amplify a fragment of the genome of the individual that includes one mutation and/or epigenetic alteration.
47. The kit of clause 44, wherein the mutation and/or epigenetic alteration can be selected from the group consisting of the mutations and/or epigenetic alteration as defined in Table 1.

## Claims

1. A method for the diagnosis and/or monitoring the recurrence of bladder cancer in a subject, comprising:
a) determining an input level of nucleic acid from a urine sample comprising nucleic acid from the subject based on data from at least one quality control assay; and
b) determining a presence or an absence of at least one mutation and/or epigenetic alteration in at least one gene associated with bladder cancer in the nucleic acid in nucleic acid sequence data obtained from an analysis of the nucleic acid in the sample, wherein the at least one gene is selected from the list of genes listed in Table 1,
wherein the at least one quality control assay comprises real time PCR analysis and/or capillary electrophoresis analysis to detect nucleic acid fragment sizes greater than 100-150 bp in size.

2. The method of claim 1, wherein obtaining nucleic acid data from the analysis of the nucleic acid in the sample comprises:
performing hybrid capture on the input nucleic acid to create a captured nucleic acid and analyzing the captured nucleic acid to obtain nucleic acid sequence data.

3. The method of claim 1 or 2, wherein the at least one quality control assay further comprises:
(i) real time PCR analysis to detect nucleic acid that is of amplifiable quality
(ii) PCR analysis to determine the relative abundance of human nucleic acid to non-human nucleic acid; or
(iii) capillary electrophoresis and fluorimetry analysis to detect double stranded nucleic acid.

4. The method of any of claims 1-3, wherein the isolated nucleic acids are analyzed directly without an amplification step.

5. The method of any of claims 1-3, wherein the isolated nucleic acid is RNA, and wherein the RNA is reverse-transcribed into complementary DNA, and wherein the reverse transcription is performed alone or in combination with an amplification step.

6. The method of claim 2 or 3, wherein the nucleic acid is amplified for enrichment of known bladder cancer genes prior to analyzing the captured nucleic acid.

7. The method of any of claim 1-3, wherein the test sample comprises one or more urine samples collected at different time points.

8. The method of any of claim 1-3, further comprising determining the presence or an absence of one or more mutations in the at least one gene associated with bladder cancer from a genotype dataset derived from the subject.

9. The method of any of claim 1-3, further comprising contacting the nucleic acid in the sample with one or more reagents suitable for detecting the presence or absence of the at least one or more mutation and/or epigenetic alteration in the one or more genes associated with bladder cancer.

10. The method of any of claim 1-3, further comprises comparing the presence or absence of the one or more mutations and/or epigenetic alterations detected in a first urine sample nucleic acid to the presence or absence of the one or more mutations and/or epigenetic alterations detected in a second urine sample nucleic acid and monitoring the recurrence of bladder cancer in the subject based on the comparison.

11. The method of any of claim 1-3, further comprising detecting the at least one mutation in the at least one gene using a hybridization assay, wherein the presence or absence of a gene mutation is determined based on the hybridization of one or more allele- specific oligonucleotide probes to one or more nucleic acid molecules in the nucleic acid sample from the subject.

12. The method of any of claim 1-3, wherein the at least one gene is FGFR3.

13. The method of any of claims 1-3, further comprising, upon determining the presence of at least one mutation and/or epigenetic alteration in the at least one gene, diagnosing and/or monitoring recurrence of bladder cancer in the subject.

14. The method of any of claims 1-3, further comprising, upon determining the presence of at least one mutation and/or epigenetic alteration in the at least one gene, monitoring bladder cancer progression in the subject.

15. The method of any of claims 1-3, further comprising, upon determining the presence of at least one mutation and/or epigenetic alteration in the at least one gene, determining susceptibility of the subject to bladder cancer.
